# EUROPÄISCHE PATENTSCHRIFT

(11) **EP 2 183 222 B1**
(45) Veröffentlichungstag und Bekanntmachung des Hinweises auf die Patenterteilung: **03.08.2011**
(21) Anmeldenummer: 08785543.3
(22) Anmeldetag: 14.08.2008
(51) Int. Cl.: C07D 235/18, C07D 277/66, A61K 31/415, A61P 3/04, C07D 401/12, C07D 409/12, C07D 417/12, A61K 31/4439, A61P 3/00

(54) **AZOLOARIN DERIVATE, VERFAHREN ZU DEREN HERSTELLUNG, DIESE VERBINDUNGEN ENTHALTENDE ARZNEIMITTEL UND DEREN VERWENDUNG**
AZOLORAINE DERIVATIVES, METHOD FOR THE PRODUCTION THEREOF, PHARMACEUTICALS CONTAINING THESE COMPOUNDS, AND THE USE THEREOF
DÉRIVÉS D'AZOLOARINE, LEURS PROCÉDÉS DE PRODUCTION, PRODUITS PHARMACEUTIQUES CONTENANT CES COMPOSÉS ET UTILISATIONS DE CES PRODUITS

(30) Priorität: 23.08.2007 EP 07291036; 25.02.2008 EP 08290181
(43) Veröffentlichungstag der Anmeldung: 12.05.2010
(73) Patentinhaber: SANOFI, 75013 Paris (FR)
(72) Erfinder: ZOLLER, Gerhard, 65926 Frankfurt am Main (DE); VOSS, Marc, Dietrich, 65926 Frankfurt am Main (DE); MATTER, Hans, 65926 Frankfurt am Main (DE); HERLING, Andreas, 65926 Frankfurt am Main (DE); PHILIPPO, Christophe, F-75013 Paris (FR); NAMANE, Claudie, F-75013 Paris (FR); SANCHEZ-ARIAS, Juan-Antonio, E-28108 Alcobendas (ES)
(74) Vertreter: Fischer, Hans-Jürgen
(86) Internationale Anmeldenummer: PCT/EP2008/006685
(87) Internationale Veröffentlichungsnummer: WO 2009/024287

(56) Entgegenhaltungen:
- WO-A-99/11627
- WO-A-03/032984
- WO-A-2006/014168
- WO-A-2007/056846
- US-A1- 2006 019 965
- ARIENTI, K. L. ET AL.: "Checkpoint Kinase Inhibitors: SAR and Radioprotective Properties of a Series of 2-Arylbenzimidazoles" J. MED. CHEM., Bd. 48, 2005, Seiten 1873-1885, XP002466182 in der Anmeldung erwähnt
- ZHAO, H. ET AL.: "Discovery of 1-(4-phenoxypiperidin-1-yl)-2-arylaminoeth anone stearoyl-CoA desaturase 1 inhibitors" BIOORGANIC & MEDICINAL CHEMISTRY LETTERS, OXFORD, GB, Bd. 17, Nr. 12, 15. Juni 2007 (2007-06-15), Seiten 3388-3391, XP022097789 ISSN: 0960-894X

## Beschreibung

Die Erfindung betrifft Azoloarin Derivate und deren physiologisch verträgliche Salze.

WO 99/11627 offenbart Phenyl-Benzimidazole mit antibakterieller Wirkung..

WO 03//032984 offenbart Aryl-Benzimidazole als Checkpoint Kinase Inhibitoren.

US 2006/019965 offenbart Benzimidazole als Gonadotropin Releasing Hormone Rezeptor Antaogonisten. Stearoyl-CoA-Desaturase-Inhibitoren sind aus WO 2006/014168 und WO 2007/056846 bekannt.

Der Erfindung lag die Aufgabe zugrunde, neue Verbindungen zur Verfügung zu stellen, die eine therapeutisch verwertbare Wirkung entfalten. Insbesondere bestand die Aufgabe darin, neue Verbindungen zu finden, die zur Behandlung von erhöhten Lipidkonzentrationen im Blut und in Geweben, dem metabolischen Syndrom, Obesitas, insbesondere viscerale (abdominale) Adipositas, einschließlich der Verhinderung der damit verbundenen Folgeerkrankungen, Diabetes, Insulinresistenz, der Dysregulation von LDL, HDL und VLDL oder Herzkreislauferkrankungen geeignet sind.

Die Erfindung betrifft daher Verbindungen der Formel I, 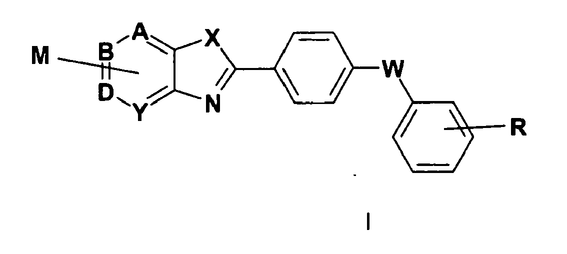 worin bedeuten
- M: R1-NH-C(=O)-;
- W: O, C=O;
- X: NH;
- A, B, D, Y: CH;
- R: (C₁-C₁₆)-Alkyl, (C₁-C₅)-Alkyloxy, (C₁-C₅)-Alkylthio, Amino, (C₁-C₅)- Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
- R1: -(CH₂)ₙ-(C₆-C₁₀)-Aryl, -(CH₂)ₙ-(C₃-C₁₂)-Heteroaryl, -(CH₂)ₙ-(C₃-C₁₂)-Hetero- cyclyl oder -(CH₂)ₙ -(C₃-C₁₂)-Cycloalkyl,
wobei Aryl-, Heteroaryl-, Heterocyclyl- oder Cycloalkylringe gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- n: 1, 2, 3, 4;
und deren physiologisch verträgliche Salze.

In einer Ausführungsform sind Verbindungen der Formel I bevorzugt, in denen W gleich O ist.

In einer Ausführungsform sind Verbindungen der Formel I bevorzugt, in denen W gleich CH₂ ist.

In einer Ausführungsform sind Verbindungen der Formel I bevorzugt, in denen W gleich C=O ist.

In einer Ausführungsform sind Verbindungen der Formel I bevorzugt, in denen X gleich N-R4 ist.

In einer Ausführungsform sind Verbindungen der Formel I bevorzugt, in denen X gleich O ist.

In einer Ausführungsform sind Verbindungen der Formel I bevorzugt, in denen X gleich S ist.

In einer Ausführungsform sind Verbindungen der Formel I bevorzugt, in denen R1 bedeutet:
- R1: (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können.

In einer Ausführungsform sind Verbindungen der Formel I bevorzugt, in denen R1 bedeutet:
(C₁-C₁₀)-Alkyl,
   wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
   wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, substituiert sein können.

In einer Ausführungsform sind Verbindungen der Formel I bevorzugt, in denen A, B, D, Y bedeuten C(R3), C(R3), C(R3), C(R3).
In einer Ausführungsform sind Verbindungen der Formel I bevorzugt, in denen A, B, D, Y bedeuten NH, C(R3), C(R3), C(R3).

In einer Ausführungsform sind Verbindungen der Formel I bevorzugt, in denen A, B, D, Y bedeuten C(R3), NH, C(R3), C(R3).

In einer Ausführungsform sind Verbindungen der Formel I bevorzugt, in denen A, B, D, Y bedeuten C(R3), C(R3), NH, C(R3).

In einer Ausführungsform sind Verbindungen der Formel I bevorzugt, in denen A, B, D, Y bedeuten C(R3), C(R3), C(R3), NH.

In einer Ausführungsform sind Verbindungen der Formel I bevorzugt, in denen A, B, D, Y bedeuten NH, NH, C(R3), C(R3).

In einer Ausführungsform sind Verbindungen der Formel I bevorzugt, in denen A, B, D, Y bedeuten C(R3), NH, NH, C(R3).

In einer Ausführungsform sind Verbindungen der Formel I bevorzugt, in denen A, B, D, Y bedeuten C(R3), C(R3), NH, NH.

In einer Ausführungsform sind Verbindungen der Formel I bevorzugt, in denen A, B, D, Y bedeuten NH, C(R3), NH, C(R3).

In einer Ausführungsform sind Verbindungen der Formel I bevorzugt, in denen A, B, D, Y bedeuten NH, C(R3), C(R3), NH.

In einer Ausführungsform sind Verbindungen der Formel I bevorzugt, in denen A, B, D, Y bedeuten C(R3), NH, C(R3), NH.

In einer Ausführungsform sind Verbindungen der Formel I bevorzugt, in denen R3 gleich Wasserstoff ist.
In einer Ausführungsform sind Verbindungen der Formel I bevorzugt, in denen R3 gleich (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)-Alkylcarbonyl, Halogen, Trifluormethyl, Trifluormethyloxy, (C₁-C₆)-Alkylsulfonyl oder Aminosulfonyl ist.

In einer Ausführungsform sind Verbindungen der Formel I bevorzugt, in denen R gleich Wasserstoff ist.

In einer Ausführungsform sind Verbindungen der Formel I bevorzugt, in denen R gleich C1 ist.

In einer Ausführungsform sind Verbindungen der Formel I bevorzugt, in denen R gleich CF₃ ist.

In einer Ausführungsform sind Verbindungen der Formel I bevorzugt, in denen M gleich R5-S(O)₀₋₂-N(R1)- oder R1-N(R2)-S(O)₀₋₂- ist.

In einer Ausführungsform sind Verbindungen der Formel I bevorzugt, in denen M ein Heterocyclus ist, der 2 bis 4 Heteroatome aus der Gruppe N, O, S enthalten kann, wobei der Heterocyclus substituiert sein kann durch (C₁-C₁₆)-Alkyl, oder doppelt gebundenen Sauerstoff.

In einer Ausführungsform sind Verbindungen der Formel I bevorzugt, in denen M gleich R1-N(R2)-C(=O)- ist.

In einer Ausführungsform sind Verbindungen der Formel I bevorzugt, in denen M gleich R5-C(=O)-N(R1)- ist.

Die Erfindung betrifft weiterhin die Verwendung der Verbindungen der Formel I, 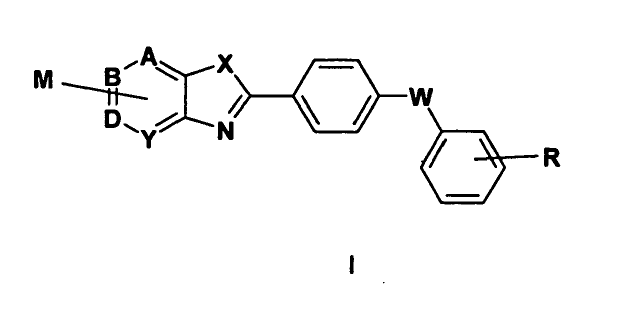 worin bedeuten
- M: R1-N(R2)-C(=O)-, R5-C(=O)-N(R1)-, R5-S(O)₀₋₂-N(R1)-, R1-N(R2)-S(O)₀₋₂-, ein Heterocyclus, der 2 bis 4 Heteroatome aus der Gruppe N, O, S enthalten kann, wobei der Heterocyclus substituiert sein kann durch (C₁-C₁₆)-Alkyl, oder doppelt gebundenen Sauerstoff;
- W: O, CH₂, C=O;
- X: N-R4, O, S;
- A, B, D, Y: unabhängig voneinander C(R3) oder N,
wobei maximal zwei der Reste A, B, D, Y die Bedeutung N haben können;
- R: Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)-Alkylsulfonyl oder Aminosulfonyl;
- R1: (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können; (C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino (C₁- C₆)-Alkylamino, Di-(C₂-C₁₂)-alkylamino, substituiert sein können;
-(C₆-C₁₀)-Aryl, -(C₃-C₁₂)-Heteroaryl, -(C₃-C₁₂)-Heterocyclyl oder -(C₃-C₁₂)- Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁-C₆)-Alkylamino, Di-(C₂- C₁₂)-alkylamino, substituiert sein können;
- R2: Wasserstoff, (C₂-C₁₆)-Alkyl, -(C₆-C₁₀)-aryl, (C₁-C₄)-Alkylen-(C₆-C₁₀)-aryl;
- oder R1 und R2: bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, - C(=O)-, -O-, -S-, -SO- und -SO₂- ersetzt sein können;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- R4: Wasserstoff, (C₁-C₅)-Alkyl;
- R5: Wasserstoff, (C₁-C₁₆)-Alkyl, Amino, (C₁-C₁₆)-Alkylamino, Di-(C₂-C₁₂)- alkylamino,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können; -(C₆-C₁₀)-Aryl, -(C₃-C₁₂)-Heteroaryl, -(C₃-C₁₂)-Heterocyclyl oder -(C₃-C₁₂)- Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁-C₆)Alkylamino, Di-(C₂- C₁₂)-alkylamino, substituiert sein können;
und deren physiologisch verträgliche Salze zur Herstellung eines Medikamentes zur Behandlung von Obesitas.

Bevorzugt ist die Verwendung der Verbindungen der Formel I, worin bedeuten
- M: R1-N(R2)-C(=O)-, R5-C(=O)-N(R1)-, R5-S(O)₀₋₂-N(R1)-, R1-N(R2)-S(O)₀₋₂-, ein Heterocyclus, der 2 bis 4 Heteroatome aus der Gruppe N, O, S enthalten kann, wobei der Heterocyclus substituiert sein kann durch (C₁-C₁₆)-Alkyl, oder doppelt gebundenen Sauerstoff;
- W: O, C=O;
- X: N-R4;
- A, B, D, Y: unabhängig voneinander C(R3) oder N,
wobei maximal zwei der Reste A, B, D, Y die Bedeutung N haben können;
- R: Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)-Alkylsulfonyl oder Aminosulfonyl;
- R1: (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl, wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₂-C₁₆)-Alkyl, -(C₆-C₁₀)-aryl, (C₁-C₄)-Alkylen-(C₆-C₁₀)-aryl;
- oder R1 und R2: bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, *- C(=O)-, -O-, -S-, -SO- und -SO₂- ersetzt sein können;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- R4: Wasserstoff, (C₁-C₅)-Alkyl
- R5: Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)-Alkylamino, Di- (C₂-C₁₂)-alkylamino, -(C₆-C₁₀)-Aryl, -(C₃-C₁₂)-Heteroaryl, -(C₃-C₁₂)-Hetero- cyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)- Alkylmercapto, Amino, (C₁-C₆)-Alkylamino, Di-(C₂-C₁₂)-alkylamino, substituiert sein können;
und deren physiologisch verträgliche Salze zur Herstellung eines Medikamentes zur Behandlung von Obesitas.

Besonders bevorzugt ist die Verwendung der Verbindungen der Formel I, worin bedeuten
- M: R1-N(R2)-C(=O)-, R5-C(=O)-N(R1)-;
- W: O, C=O;
- X: N-R4;
- A, B, D, Y: unabhängig voneinander C(R3) oder N,
wobei maximal zwei der Reste A, B, D, Y die Bedeutung N haben können;
- R: Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)-Alkylsulfonyl oder Aminosulfonyl;
- R1: (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl,- (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₂-C₁₆)-Alkyl, -(C₆-C₁₀)-aryl, (C₁-C₄)-Alkylen-(C₆-C₁₀)-aryl;
- oder R1 und R2: bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, - C(=O)-, -O-, -S-, -SO- und -SO₂- ersetzt sein können;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- R4: Wasserstoff, (C₁-C₅)-Alkyl
- R5: Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
und deren physiologisch verträgliche Salze zur Herstellung eines Medikamentes zur Behandlung von Obesitas.

Ganz besonders bevorzugt ist die Verwendung der Verbindungen der Formel I, worin bedeuten
- M: R1-N(R2)-C(=O)-, R5-C(=O)-N(R1)-;
- W: O, C=O;
- X: N-R4;
- A, B, D, Y: CH;
- R: Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)-Alkylsulfonyl oder Aminosulfonyl;
- R1: (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₂-C₁₆)-Alkyl, -(C₆-C₁₀)-aryl, (C₁-C₄)-Alkylen-(C₆-C₁₀)-aryl;
oder R1 und R2 bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, - CR4R5-, -(C=R4)-, -NR6-, -C(=O)-, -O-, -S-, -SO- und -SO₂- ersetzt sein können;
- R4: Wasserstoff;
- R5: Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
und deren physiologisch verträgliche Salze zur Herstellung eines Medikamentes zur Behandlung von Obesitas.

Die Erfindung betrifft Verbindungen der Formel I in Form ihrer Salze, Racemate, optische Isomere, Rotationsisomere, racemischen Mischungen und reinen Enantiomere, und deren Diastereomere und Gemische davon. Die Trennung der Gemische erfolgt auf chromatographischem Weg.

Die vorliegende Erfindung umfasst alle möglichen tautomeren Formen der Verbindungen der Formel I.

Die vorliegende Erfindung umfasst weiterhin Derivate der Verbindungen der Formel I, zum Beispiel Solvate, wie Hydrate und Alkoholaddukte, Ester, Prodrugs und andere physiologisch akzeptablen Derivate der Verbindungen der Formel I, sowie aktive Metaboliten der Verbindungen der Formel I. Die Erfindung umfasst ebenfalls alle Kristallmodifikationen der Verbindungen der Formel I.

Die Alkylreste in den Substituenten R, R1, R2, und R3 können entweder geradkettig oder verzweigt sein.

Halogen steht für Fluor, Chlor, Brom oder Iod, insbesondere Brom oder Chlor.

Aryl steht für einen monocyclischen oder bicyclischen aromatischen Kohlenwasserstoffrest mit 6 bis 10 Ringatomen, der unabhängig durch einen bis vier, vorzugsweise einen oder zwei, beschriebene Substituenten substituiert sein kann.

Heteroaryl steht für einen monocyclischen oder bicyclischen Rest mit 5 bis 12 Ringatomen mit wenigstens einem aromatischen Ring mit einem, zwei oder drei Ringheteroatomen ausgewählt aus N, O und S, wobei die restlichen Ringatome C sind.
Cycloalkyl steht für ein gesättigtes oder teilweise ungesättigtes Ringsystem (das ausschließlich Kohlenstoffatome enthält), das ein oder mehrere Ringe umfaßt.

Heterocyclyl steht für ein gesättigtes oder teilweise ungesättigtes Ringsystem (das wenigstens ein Heteroatom enthält), das ein oder mehrere Ringe umfaßt.

Bicyclyl steht für ein bicyclisches gesättigtes oder teilweise ungesättigtes Ringsystem, wobei die einzelnen Glieder der Ringsysteme ausschließlich Kohlenstoffatome oder ein, zwei oder drei Ringheteroatome ausgewählt aus N, O und S enthalten können, wobei die restlichen Ringatome C sind. Bei einem der Ringe in dem bicyclischen System kann es sich auch um einen kondensierten aromatischen Ring wie Benzol handeln

Können Reste oder Substituenten mehrfach in den Verbindungen der Formel I auftreten (wie zum Beispiel "R3"), so können sie alle unabhängig voneinander die angegebenen Bedeutungen haben und gleich oder verschieden sein.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfam- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon-, und Weinsäure. Für medizinische Zwecke wird in besonders bevorzugter Weise das Chlorsalz verwendet. Geeignete physiologisch verträgliche basische Salze sind Ammoniumsalze, Alkalimetallsalze (wie Natrium- und Kaliumsalze), Erdalkalisalze (wie Magnesium- und Calciumsalze), Zinksalze, Trometamol (2-Amino-2-hydroxymethyl-1,3-propandiol)-, Diethanolamin-, Lysin-, Arginin-, Cholin-, Meglumin- oder Ethylendiamin-Salze.

Salze mit einem nicht physiologisch verträglichen Anion oder Kation gehören ebenfalls in den Rahmen der Erfindung als nützliche Zwischenprodukte für die Herstellung oder Reinigung physiologisch verträglicher Salze und/oder für die Verwendung in nicht-therapeutischen, zum Beispiel in-vitro-Anwendungen.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und wie hierin beschrieben.

Die Verbindungen der Formel I und deren physiologisch verträgliche Salze und physiologisch funktionelle Derivate stellen ideale Arzneimittel zur Behandlung von erhöhten Lipidkonzentrationen im Blut und in Geweben, dem metabolischen Syndrom, Obesitas, Diabetes, Insulinresistenz, der Dysregulation von LDL, HDL und VLDL oder Herzkreislauferkrankungen Lipidstoffwechselstörungen, insbesondere von Hyperlipidämie dar. Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Kombinationen mit anderen Medikamenten

Die erfindungsgemäßen Verbindungen können alleine oder in Kombination mit einem oder mehreren weiteren pharmakologischen Wirkstoffen, die beispielsweise günstige Wirkungen auf Stoffwechselstörungen bzw. häufig damit assoziierten Erkrankungen haven, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.

Als weitere Wirkstoffe für die Kombinationspräparate sind insbesonders geeignet: Alle Antidiabetika, die in der Roten Liste 2006, Kapitel 12 genannt sind; alle Abmagerungsmittel/Appetitzügler, die in der Roten Liste 2005, Kapitel 1 gennant sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn ™ (Generex Biotechnology), GLP-1-Derivate wie z.B. Exenatide, Liraglutide oder diejenigen die in WO 98/08871, WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe,
Biguanide,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11β-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., W02005021497) oder mit Verbindungen, wie in WO2002066464 (Kotobuki Pharmaceutical Co. Ltd.) oder WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit PPAR alpha Agonist, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhihitor, wie z.B. Torcetrapib oder JTT-705 , verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in W02005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht. Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, W02005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031 oder WO2004072066 oder WO2005080360 beschrieben sind, verabreicht.

Bei einer Ausführungsform wird die Verbindung der Formel I in Kombination mit einem Inhibitor der Glukoneogenese, wie z. B. FR-225654, verabreicht.
Bei einer Ausführungsform wird die Verbindung der Formel I in Kombination mit Inhibitoren der Fructose-1,6-bisphosphatase (FBPase) wie z.B. CS-917, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733 oder solche, wie sie z. B. in WO200190090-94, WO200343999, W02004112782, W0200344000, W0200344009, W02004112779, W02004113310, WO2004103980, W02004112784, W02003065983, WO2003104207, WO2003104208, WO2004106294, W02004011410, W02004033427, WO2004041264, WO2004037251, WO2004056744, WO2004065351, WO2004089367, WO2004089380, WO2004089470-71, WO2004089896, W02005016877, WO2005097759 beschrieben sind, verabreicht.

Bei einer Ausführungsform wird die Verbindung der Formel I in Kombination mit Inhibitoren der Protein-Tyrosin-Phosphatase-1B (PTP 1 B), wie sie z. B. in W0200119830-31, W0200117516, W02004506446, WO2005012295, PCT/EP2005/005311, PCT/EP2005/005321, PCT/EP2005/007151 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, W02005085230, PCT/EP2005/005346, WO2003078403, WO2004022544, WO2003106410, W02005058908, US2005038023, WO2005009997, US2005026984, WO2005000836, W02004106343, EP1460075, W02004014910, WO2003076442, WO2005087727, W02004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht. Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in W02001000610, W02001030774, W02004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
CB1R (Cannabinoid Rezeptor 1) Antagonisten (wie z.B. Rimonabant, SR147778, SLV-319, AVE-1625, MK-0364 oder Salze davon oder solche wie sie in z. B. EP 0656354, WO 00/15609, WO2001/64632, WO2001/64633, WO2001/64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, W02004058145, WO2003084930, WO2003084943, WO2004058744, W02004013120, WO2004029204, WO2004035566, W02004058249, WO2004058255, WO2004058727, W02004069838, US20040214837, US20040214855, US20040214856, WO2004096209, WO2004096763, WO2004096794, WO2005000809, WO2004099157, US20040266845, W02004110453, WO2004108728, WO2004000817, WO2005000820, US20050009870, WO200500974, WO2004111033-34, WO200411038-39, WO2005016286, W02005007111, WO2005007628, US20050054679, WO2005027837, WO2005028456, WO2005063761-62, W02005061509, WO2005077897, WO2006047516, WO2006060461, WO2006067428, W02006067443 beschrieben sind);

MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, W02004087159, WO2004078717, WO2004078716, WO2004024720, US20050124652, WO2005051391, W02004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, W02004011438, WO2004012648, WO2003015769, WO2004072025, WO2005070898, WO2005070925, WO2004039780, W02003033476, W02002006245, WO2002002744, WO2003004027, FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356 oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-düsopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. in US2004/0224997, W02004094618, W0200058491, WO2005044250, W02005072740, JP2005206492, WO2005013907 beschrieben;
Inhibitoren der Fettsäuresynthase (FAS) wie z.B. C75 oder solchen, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, W02004018421, W02005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

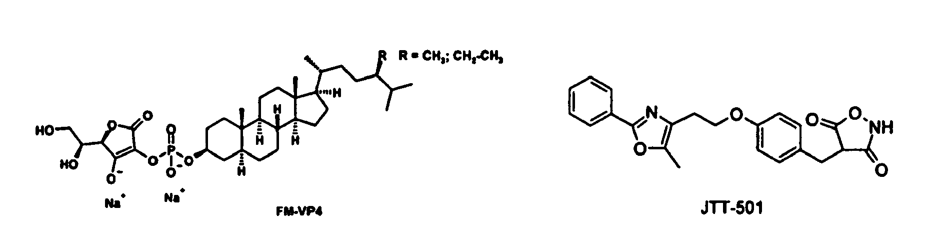

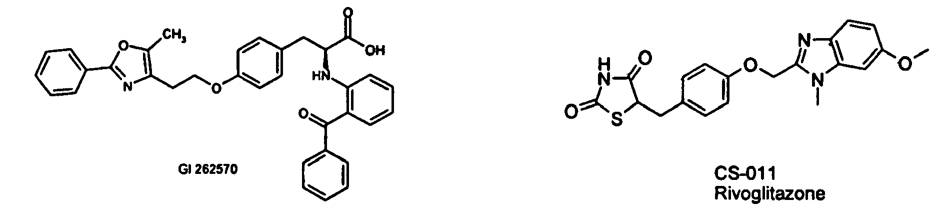

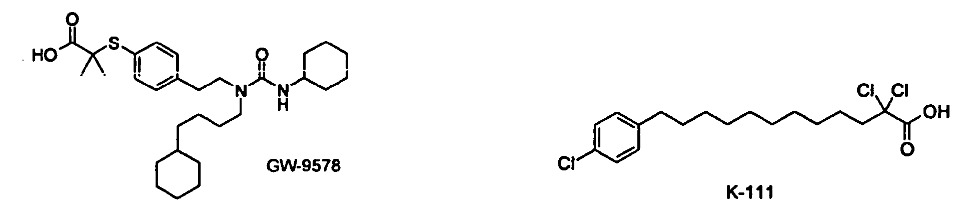

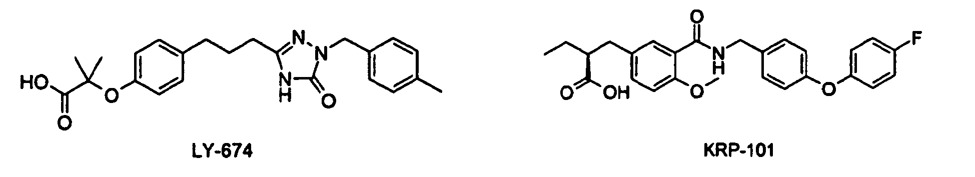

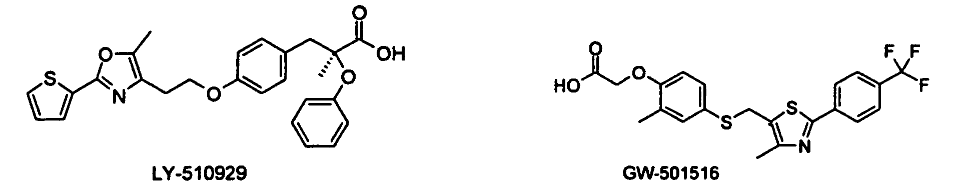

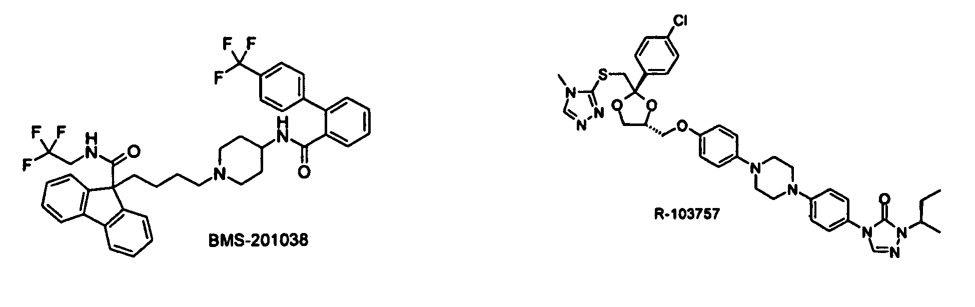

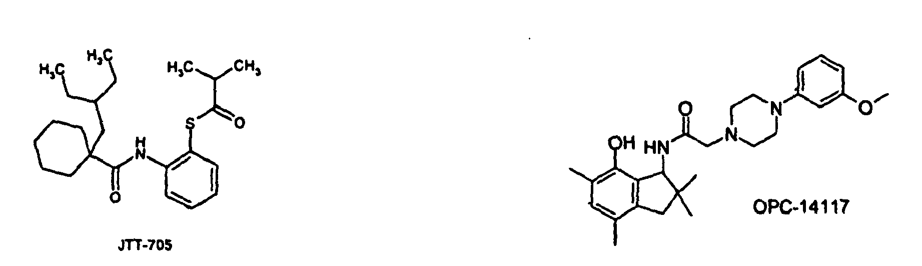

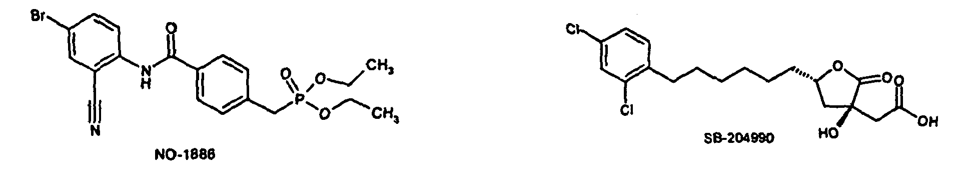

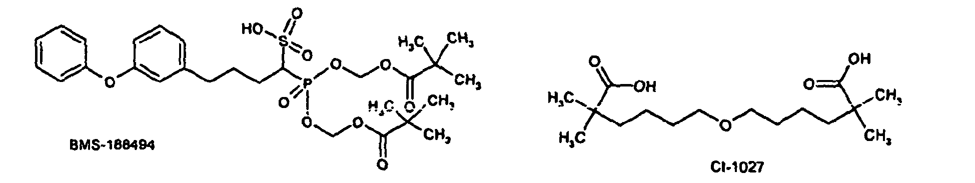

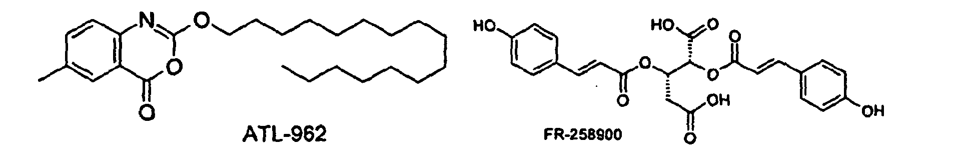

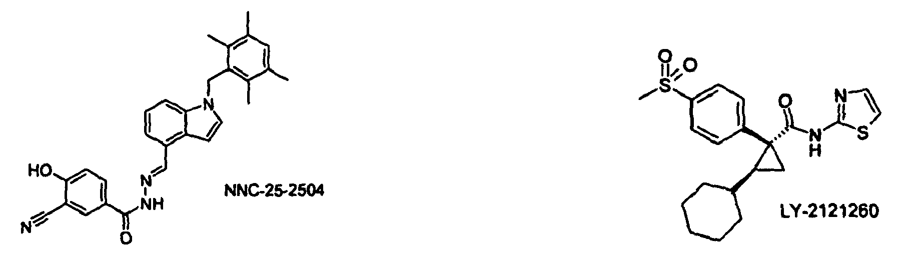

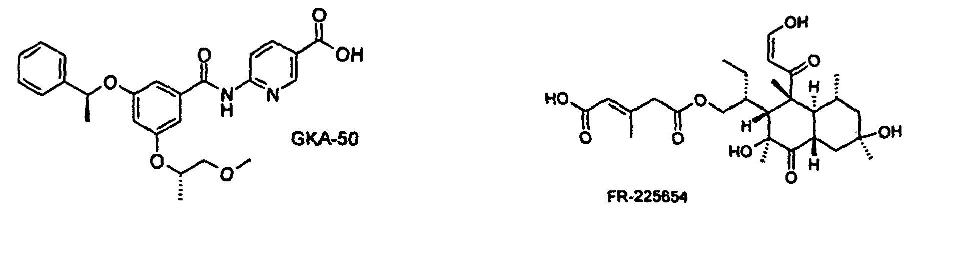

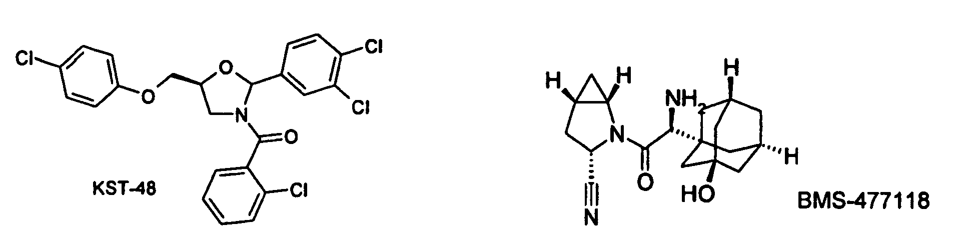

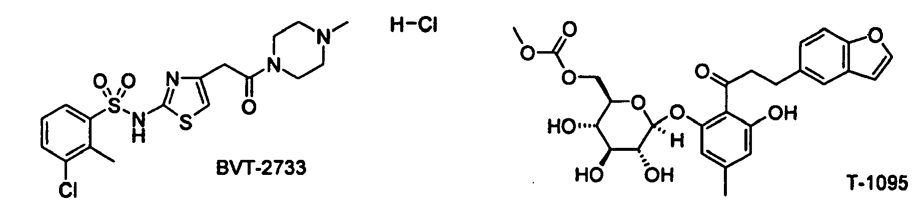

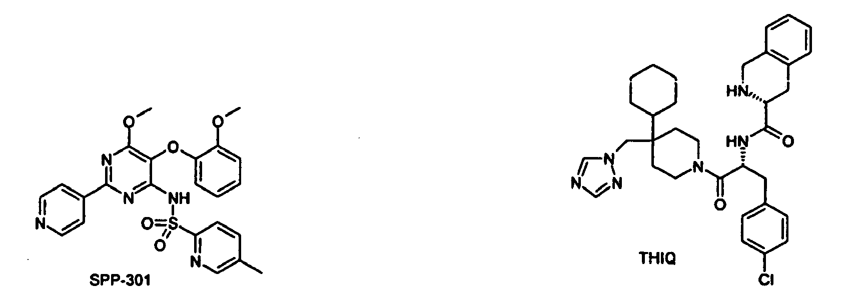

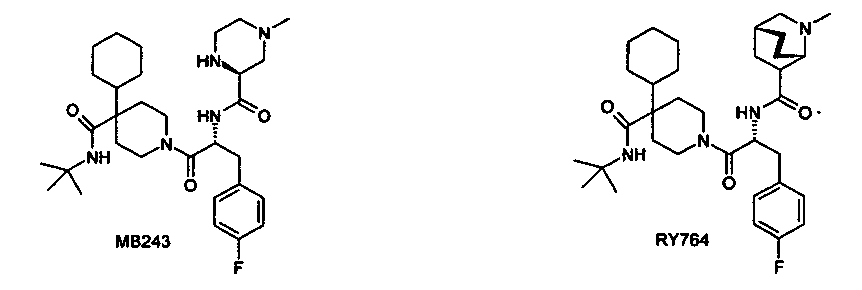

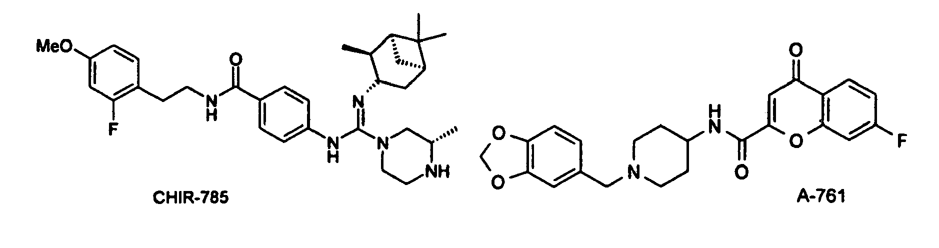

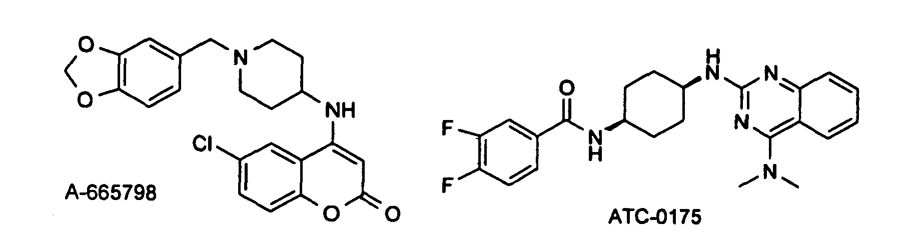

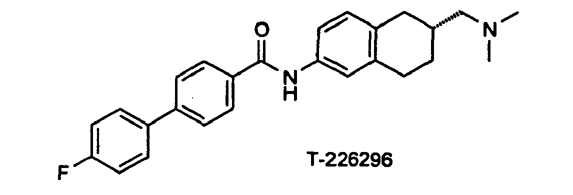

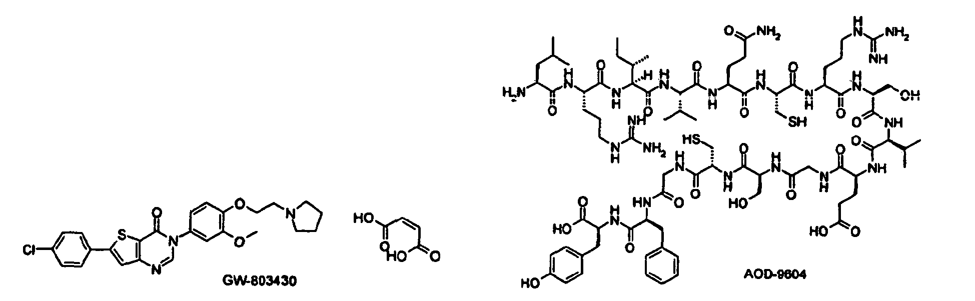

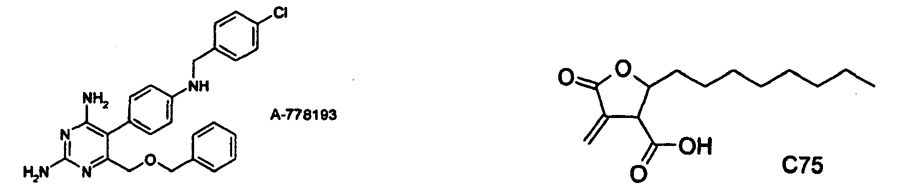

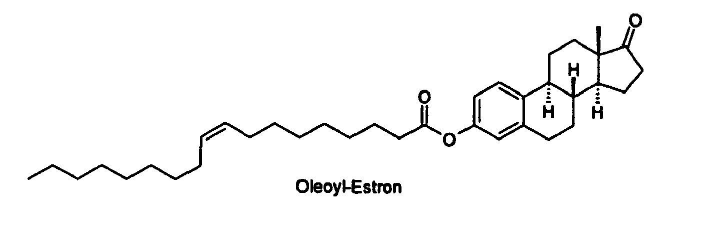

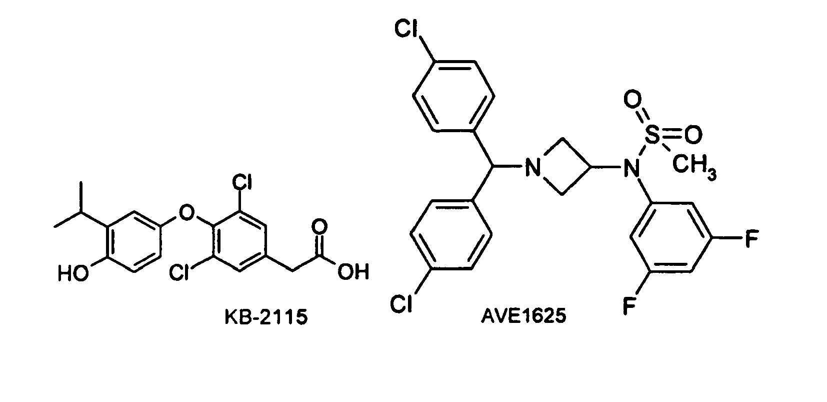

Die nachfolgenden Beispiele dienen zur näheren Erläuterung der Erfindung, ohne dieselbe auf in den Beispielen beschriebene Produkte und Ausführungsformen einzuschränken.

**Tabelle 1:**

| | | | | | | | | |
|---|---|---|---|---|---|---|---|---|
| 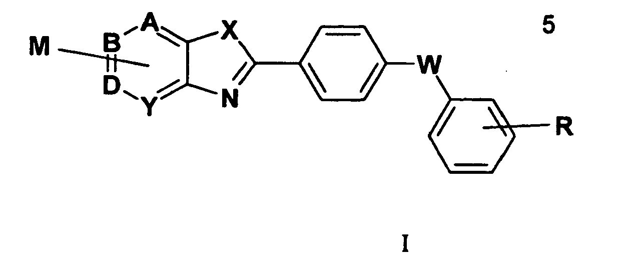 | | | | | | | | |

| **Bsp** | **R** | **W** | **X** | **A** | **B** | **D** | **Y** | **M [Bindungsstelle an Atom]** |
|---|---|---|---|---|---|---|---|---|
| 2 | H | O | NH | CH | CH | CH | CH | CONHCH₂CH₂CH₂-(tryridyl-3-yl); [B] |
| 3 | | H O | NH | CH | CH | CH | CH | CONHCH₂CH₂CH₂-phenyl; [B] |
| 4 | H | O | NH | CH | CH | CH | CH | CONHCH₂CH₂-phenyl; [B] |
| 5 | H | O | NH | CH | CH | CH | CH | CONHCH₂CH₂CH(CH₃)₂; [B] |
| 6 | H | O | NH | CH | CH | CH | CH | CONHCH₂CH₂-cyclopropyl; [B] |
| 7 | H | O | NH | CH | CH | CH | CH | CONHCH₂-cyclopropyl; [B] |
| 8 | H | O | NH | CH | CH | CH | CH | CONHCH₂-(5-chlorthiophen-2-yl); [B] |
| 9 | H | O | NH | CH | CH | CH | CH | CONHCH₂-(thiazol-2-yl); [B] |
| 10 | H | O | NH | CH | CH | CH | CH | CONHCH₂CH₂CH₂-O-CH₂CH₂CH₂CH₃; [B] |
| 12 | 2-Cl | O | NH | CH | CH | CH | CH | CONHCH₂CH₂-phenyl; [B] |
| 13 | 2-Cl | O | NH | CH | CH | CH | CH | CONHCH₂CH₂CH(CH₃)₂; [B] |
| 14 | 2-Cl | O | NH | CH | CH | CH | CH | CONHCH₂CH₂-cyclopropyl; [B] |
| 15 | 2-Cl | O | NH | CH | CH | CH | CH | CONHCH₂-(5-chlorthiophen-2-yl); [B] |
| 16 | 2-Cl | O | NH | CH | CH | CH | CH | CONHCH₂-(thiazol-2-yl); [B] |
| 18 | 2-CF₃ | O | NH | CH | CH | CH | CH | CONHCH₂-(5-chlorthiophen-2-yl); [D] |
| 19 | 2-CF₃ | CO | NH | CH | CH | CH | CH | CONHCH₂-(thiophen-2-yl); [B] |
| 20 | 2-CF₃ | CO | NH | CH | CH | CH | CH | CONHCH₂CH(CH₃)₂; [B] |
| 22 | 2-CF₃ | O | S | CH | CH | CH | CH | CONHCH₂CH(CH₃)₂; [D] |
| 23 | 2-CF₃ | O | NH | CH | CH | CH | CH | CONHCH₂CH₂-cyclopropyl; [D] |
| 24 | 2-CF₃ | O | NH | CH | CH | CH | CH | CONHCH₂CH(CH₃)₂; [D] |
| 25 | 2-CF₃ | O | NH | CH | CH | CH | CH | CONHCH₂-(thiazol-2-yl); [D] |
| 26 | 2-CF₃ | O | NH | CH | CH | CH | CH | CONHCH₂CH₂CH₂-phenyl; [D] |
| 27 | 2-CF₃ | CO | NH | CH | CH | CH | CH | CONHCH₂-(5-chlorthiophen-2-yl); [D] |
| 28 | 2-CF₃ | CO | NH | CH | CH | CH | CH | CONHCH₂-(thiazol-2-yl); [B] |

Die Wirksamkeit der erfindungsgemäßen Verbindungen der Formel I wurde in dem folgenden Assaysystem mit mikrosomalen Enzymen untersucht:

Die Lebern von männlichen Wistar-Ratten, die (zur Induktion der Expression von SCD1) mit einer kohlenhydratreichen, fettarmen Nahrung gefüttert worden waren, wurden mit einem Potter-Gewebehomogenisator und Differentialzentifugation in einem Puffer homogenisiert, der 250 mM/l Saccharose und 5 mM HEPES (pH 7,0) enthielt. Die resuspendierte Mikrosomenfraktion wurde bei -80°C aufbewahrt. Die Stearoyl-CoA-Desaturase-Aktivität wurde in einem Dünnschichtchromatographieassay mit 1-¹⁴C-markierter Stearinsäure bestimmt. Kurz gesagt wurden die jeweiligen erfindungsgemäßen Verbindungen (in DMSO in einer Endkonzentration von 1% [v/v]) mit 15 µg der Rattenlebermikrosomen in 200µl Assaypuffer (6 mM MgCl₂, 250 mM Saccharose, 150 mM KCl, 40 mM NaF, 100 mM Na₂HPO₄(pH7,4), 1,3 mM ATP, 1,5 mM reduziertes Glutathion, 60 uM CoA, 0,33 mM Nicotinamid und 0,94 mM NADH) 10 min bei Raumtemperatur inkubiert. 0,5 µCi [1-¹⁴C]-Stearinsäure (55 mCi/mmol) wurden zugegeben, und die Mischung wurde 1 h bei 37°C inkubiert. Die radioaktiv markierten Fettsäuren wurden anschließend bei 65°C 4 h mit 2,5 M KOH/MeOH:H₂0(4:1) verseift, mit 280 µl Ameisensäure protoniert und mit 500 µl Hexan extrahiert. Die DC-Platten wurden in 10%ige AgNO₃ eingetaucht und vor der Verwendung hitzeaktiviert. 150 µl der Hexanphase wurden auf die Platten aufgebracht, und die DC-Platten wurden in Puffer (Chloroform:Methanol:Essigsäure:Wasser [90:8:1:0,8]) entwickelt und getrocknet. Die Platten wurden zur Quantifizierung der SCD1-Aktivität in einem Phospho-Imager abgelesen.

Der Fachmann kann diesen Assay zur Bestimmung der Inhibierung der Stearoyl-CoA-Desaturase-Aktivität in verschiedener Hinsicht modifizieren. Repräsentative erfindungsgemäße Verbindungen wie die in den Beispielen beschriebenen zeigten, wenn sie in diesem Assay in einer Konzentration von 10 µM getestet wurden, Aktivität als Inhibitoren von SCD1, die als Hemmung der SCD1-Aktivität in Prozent angegeben ist.

**Tabelle 2: Biologische Aktivität ausgewählter Verbindungen**

| Bsp. | % Inhibition (10 µM) |
|---|---|
| 8 | 37 |
| 9 | 29 |
| 13 | 58 |
| 14 | 80 |
| 15 | 95 |
| 16 | 86 |
| 19 | 100 |
| 20 | 77 |
| 23 | 96 |
| 24 | 77 |
| 25 | 100 |
| 27 | 100 |
| 28 | 100 |

Die Verbindungen der Formel I hemmen die SCD1-Aktivität und sind daher gut zur Behandlung von Fettstoffwechselstörungen, Obesitas und dem metabolischen Syndrom geignet (Hulver et al. Cell Metabolism (2005), 2(4), 251-261 sowie Warensjoe et al. Diabetologia (2005), 48(10), 1999-2005).

Aufgrund der Hemmung der SCD-Aktivität können die Verbindungen der Formel I auch zur Behandlung bzw. Prävention weiterer durch SCD vermittelten Krankheiten bzw. eines durch SCD vermittelten Leidens in einem Säugetier, vorzugsweise einem Menschen, angewendet werden.
Die Verbindungen der vorliegenden Erfindung eignen sich insbesondere zur Behandlung und/oder Prävention von:
1.
   - Obesitas, insbesondere viscerale (abdominale) Adipositas
2.
   - Störungen des Fettsäurestoffwechsels und Glucoseverwertungsstörungen
   - Störungen, bei denen Insulinresistenz eine Rolle spielt
3.
   - Diabetes mellitus, insbesondere Typ-2-Diabetes, einschließlich der Prävention der damit verbundenen Folgeerkrankungen.
   - Besondere Aspekte in diesem Zusammenhang sind
   - Hyperglykämia,
   - Verbesserung der Insulinresistenz,
   - Verbesserung der Glucosetoleranz,
   - Schutz der ß-Zellen der Bauchspeicheldrüse
   - Prävention makro- und mikrovaskulärer Erkrankungen
4. Dyslipidämien und deren Folgen wie beispielsweise Atherosklerose, koronare Herzkrankheit, zerebrovaskuläre Erkrankungen usw., insbesondere (jedoch nicht darauf beschränkt) die, die durch einen oder mehrere der folgenden Faktoren gekennzeichnet sind:
   - hohe Plasmatriglyceridkonzentrationen, hohe postprandiale Plasmatriglyceridkonzentrationen,
   - niedrige HDL-Cholesterinkonzentration
   - niedrige ApoA-Lipoproteinkonzentrationen
   - hohe LDL-Cholesterinkonzentrationen
   - kleine dichte LDL-Cholesterinpartikel
   - hohe ApoB-Lipoproteinkonzentrationen
   - Desaturierungsindex (z.B. Verhältnis 18:1 / 18:0n-9, 16:1/16:0 n-7 oder 18:1n-9 + 16:1n-7/16:0 Fettsäuren)
5. Verschiedene andere leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
6. Leberstörungen und damit in Zusammenhang stehende Leiden
   - Fettleber
   - hepatische Steatose
   - nicht alkoholbedingte Hepatitis
   - nicht alkoholbedingte Steatohepatitis (NASH)
   - alkoholbedingte Hepatitis
   - akute Fettleber
   - schwangerschaftsbedingte Fettleber
   - arzneimittelinduzierte Hepatitis
   - Eisenspeicherkrankheiten
   - hepatische Fibrose
   - hepatische Zirrhose
   - Hepatom
   - virale Hepatitis
7. Hauterkrankungen und -leiden bzw. solche, die mit mehrfach ungesättigten Fettsäuren in Zusammenhang stehen
   - Ekzem
   - Akne
   - Schuppenflechte
   - Bildung bzw. Prävention von Wulstnarben
   - andere mit der Fettsäurezusammensetzung der Schleimhautmembran in Zusammenhang stehende Krankheiten
8. Primäre Hypertriglyceridämie oder sekundäre Hypertriglyceridämien nach
   - familiärer Retikulohistiozytose
   - Lipoproteinlipasemangel
   - Hyperlipoproteinämien
   - Apolipoproteinmangel (z.B. ApoCII- oder ApoE-Mangel)
9. Krankheiten bzw. Leiden, die mit neoplastischer zellulärer Proliferation in Zusammenhang stehen
   - gutartige oder maligne Tumore
   - Krebs
   - Neoplasien
   - Metastasen
   - Karzinogenese
10. Krankheiten bzw. Leiden, die mit neurologischen, psychiatrischen oder Immunerkrankungen bzw. -leiden in Zusammenhang stehen
11. Andere Krankheiten bzw. Leiden, an denen beispielsweise entzündliche Reaktionen, Zelldifferenzierung und/oder andere durch SCD vermittelte Aspekte beteiligt sein können, sind:
   - Atherosklerose wie beispielsweise (jedoch nicht darauf beschränkt) Koronarsklerose einschließlich Angina pectoris oder Herzinfarkt, Schlaganfall, ischämischer Schlaganfall und transitorische ischämische Attacke (transient ischemic attack, TIA)
   - periphere Verschlußkrankheit
   - vaskuläre Restenose oder Reokklusion
   - chronische entzündliche Darmerkrankungen wie beispielsweise Morbus Crohn und Colitis ulcerosa
   - Pankreatitis
   - Sinusitis
   - andere entzündliche Leiden
   - Retinopathie, ischämische Retinopathie
   - Fettzellentumore
   - lipomatöse Karzinome wie beispielsweise Liposarkome
   - feste Tumore und Neoplasmen wie beispielsweise (jedoch nicht darauf beschränkt) Karzinome des Magen-Darm-Trakts, der Leber, der Gallenwege und der Bauchspeicheldrüse, endokrine Tumore, Karzinome der Lunge, der Niere und der ableitenden Harnwege, des Genitaltrakts, Prostatakarzinome usw.
   - akute und chronische myeloproliferative Erkrankungen und Lymphome
   - Angiogenese
   - neurodegenerative Erkrankungen
   - Alzheimer-Krankheit
   - multiple Sklerose
   - Parkinson-Krankheit
   - erythematosquamöse Dermatosen wie beispielsweise Schuppenflechte
   - Acne vulgaris
   - andere Hauterkrankungen und dermatologische Leiden, die durch PPAR moduliert werden
   - Ekzeme und Newodenmatitis
   - Dermatitis wie beispielsweise seborrhoische Dermatitis oder Lichtdermatitis
   - Keratitis und Keratosen wie beispielsweise seborrhoische Keratosen, senile Keratosen, aktinische Keratoses, lichtinduzierte Keratosen oder Keratosis follicularis
   - Wulstnarben und Wulstnarbenprophylaxe
   - Warzen einschließlich Kondylomen oder Condylomata acuminata
   - Infektionen mit dem humanen Papillomavirus (HPV) wie beispielsweise venerische Papillome, von Viren verursachte Warzen wie beispielsweise Molluscum contagiosum, Leukoplakie
   - papulöse Dermatosen wie beispielsweise Lichen planus
   - Hautkrebs wie beispielsweise Basalzellenkarzinome, Melanome oder kutane T-Zellenlymphome
   - lokalisierte gutartige epidermale Tumore wie beispielsweise Keratoderma, epidermale Naevi
   - Frostbeulen
   - Bluthochdruck
   - Syndrom X
   - Symdrom der polyzystischen Ovarien (polycystic ovary syndrome, PCOS)
   - Asthma
   - zystische Fibrose
   - Osteoarthritis
   - Lupus erythematodes (LE) oder entzündliche rheumatische Erkrankungen wie beispielsweise rheumatoide Arthritis
   - Gefäßentzündung
   - Auszehrung (Kachexie)
   - Gicht
   - Ischämia/Reperfusionssyndrom
   - Schocklunge (acute respiratory distress syndrome, ARDS)
   - Virenerkrankungen und -infektionen
   - Lipodystrophie und lipodystrophische Leiden, auch bei Arzneimittelnebenwirkungen (z.B. nach der Einnahme von Medikamenten zur Behandlung von HIV oder Tumoren)
   - Myopathien und Lipidmyopathien (wie Carnitinpalmitoyltransferase-I- oder -II-Mangel) Bildung von Muskeln und einem schlanken Körper bzw. Muskelmassebildung in der Tierhaltung und beim Menschen

### Darstellung

Die erfindungsgemäßen Verbindungen der allgemeinen Formel I werden durch an sich in der Literatur bekannte Verfahren (z.B. McClure, Kelly J.; Huang, Liming; Arienti, Kristen L.; Axe, Frank U.; Brunmark, Anders; Blevitt, Jon; Guy Breitenbucher, J.; Bioorganic & Medicinal Chemistry Letters (2006), 16(7), 1924-1928; Arienti, Kristen L.; Brunmark, Anders; Axe, Frank U.; McClure, Kelly; Lee, Alice; Blevitt, Jon; Neff, Danielle K.; Huang, Liming; Crawford, Shelby; Pandit, Chennagiri R.; Karlsson, Lars; Breitenbucher, J. Guy, Journal of Medicinal Chemistry (2005), 48(6), 1873-1885) dargestellt und sind nach folgenden Reaktionssequenzen erhältlich, worin die Reste die oben angegeben Bedeutungen haben.

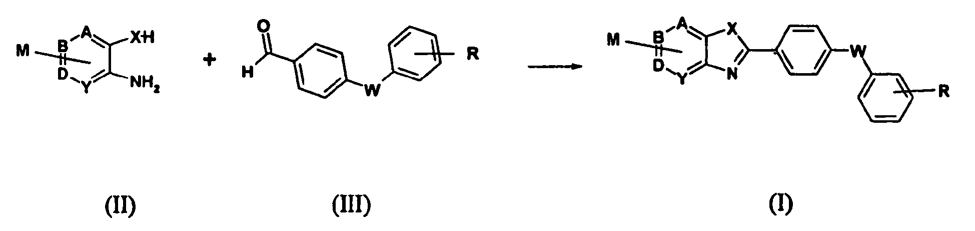

Die als Ausgangsmaterialien verwendeten Verbindungen sind kommerziell erhältlich oder lassen sich nach aus der Literatur bekannten Verfahren herstellen; 4-Phenoxy substituierte Benzaldehyde beispielsweise sind durch Umsetzung entsprechend substituierten Phenole mit 4-Fluor- oder 4-Chlorbenzaldehyd in Gegenwart einer Base erhältlich (z.B. Pfefferkorn, Jeffrey A.; Greene, Meredith L.; Nugent, Richard A.; Gross, Rebecca J.; Mitchell, Mark A.; Finzel, Barry C.; Harris, Melissa S.; Wells, Peter A.; Shelly, John A.; Anstadt, Robert A.; Kilkuskie, Robert E.; Kopta, Laurice A.; Schwende, Francis J.; Bioorganic & Medicinal Chemistry Letters (2005), 15(10), 2481-2486).
Andererseits lassen sich diese Verbindungen auch herstellen durch Umsetzung von 4-Bromphenol mit Halogenaromaten und anschließender Überführung in die entsprechenden Aldehyde.

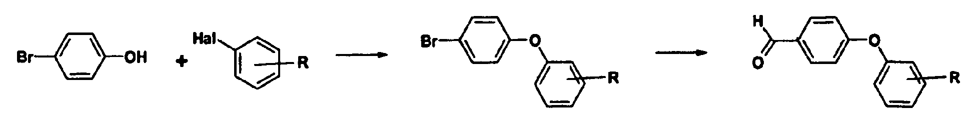

Diaminopyridin-carbonsäureester können nach P. Chand et. al, Bioorganic & Medicinal Chemistry Letters (2005), 13(17), 2665-2678 hergestellt werden.
Substituierte 4-Benzyl-benzaldehyde können analog Langle, Sandrine; Abarbri, Mohamed; Duchene, Alain, Tetrahedron Letters (2003), 44(52), 9255-9258 hergestellt werden. Benzoylsubstituierte Benzaldehyde lassen sich durch Oxydation der entsprechenden Alkohole herstellen. Diese Methode ist beschrieben in Kashiwagi, Yoshitomo; Ikezoe, Hiroshi; Ono, Tetsuya. Synlett (2006), (1), 69-72; Smith, Amos B., III; Rucker, Paul V.; Brouard, Ignacio; Freeze, B. Scott; Xia, Shujun; Horwitz, Susan Band. Organic Letters (2005), 7(23), 5199-5202.

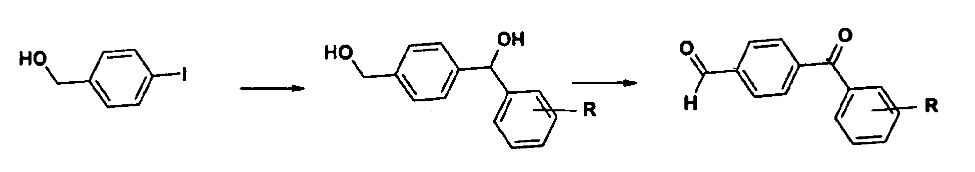

Eine alternative Methode verwendet die Friedel-Crafts-Acylierung und anschließende Seitenkettenhalogenierung und Hydrolyse: Nakatani, Kazuhiko; Dohno, Chikara; Saito, Isao. Journal of Organic Chemistry (1999), 64(18), 6901-6904.

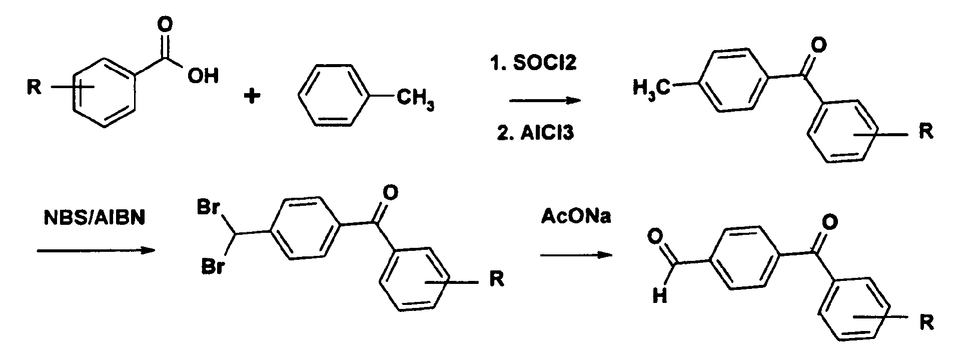

Falls bei diesen Umsetzungen Säuren freigesetzt werden, ist es vorteilhaft, zur Beschleunigung Basen wie Pyridin, Triethylamin, Natronlauge oder Alkalicarbonate zuzusetzen. Die Umsetzungen können in weiten Temperaturbereichen durchgeführt werden. Es hat sich als vorteilhaft erwiesen, bei Temperaturen von 0°C bis zum Siedepunkt des verwendeten Lösungsmittels zu arbeiten. Beispiele von verwendeten Lösungsmitteln sind Methylenchlorid, THF, DMF, Toluol, Essigsäureethylester, n-Heptan, Dioxan, Diethylether oder Pyridin. Unter wasserfreien Bedingungen haben sich auch starke Basen wie Lithiumhydrid, Natriumhydrid oder Kalium-tert.-butanolat in aprotischen Lösungsmitteln wie THF oder DMF als geeignet erwiesen.

Die Verbindungen der allgemeinen Formel I werden aus dem Reaktionsgemisch isoliert und nach an sich bekannten Verfahren wie Extraktion, Kristallisation oder Chromatographie aufgereinigt.

Die nachfolgend aufgeführten Beispiele dienen zur Erläuterung der Erfindung, ohne diese jedoch einzuschränken.
Die Identität der Verbindungen wurde durch Massenspektrometrie geprüft.

### Beispiel 1:

### 2-(4-Phenoxy-phenyl)-3H-benzimidazol-5-carbonsäureethylester

Zu einer Lösung von 4-Phenoxybenzaldehyd (11,0 g, 55,49 mmol) in 250 ml Ethanol wurden unter Argon Natriummetabisulfit (10,55 g, 55,49 mmol) in 50 ml Wasser zugetropft. Der ausfallende, dicke Niederschlag wurde mit 50 ml Ethanol verdünnt, 4 h bei Raumtemperatur gerührt und über Nacht im Kühlschrank aufbewahrt. Der Niederschlag wurde abgesaugt, mit Ethanol gewaschen und getrocknet. Das isolierte Zwischenprodukt wurde in 100 ml DMF suspendiert und nach Zugabe von 3,4-Diaminobenzoesäureethylester (10,0 g, 55,49 mmol) 4 h bei 130 °C gerührt. DMF wurde im Vakuum abdestilliert, der Rückstand mit Wasser und Essigsäureethylester versetzt, die organisch Phase abgetrennt, mit Wasser gewaschen, getrocknet und eingeengt. Das erhaltene Rohprodukt (18,3 g) wurde weiter umgesetzt.

### Beispiel 2:

### 2-(4-Phenoxy-phenyl)-3H-benzimidazol-5-carbonsäure-(3-pyridin-3-yl-propyl)-amid

### 2a: 2-(4-Phenoxy-phenyl)-3H-benzimidazol-5-carbonsäure

2-(4-Phenoxy-phenyl)-3H-benzimidazol-5-carbonsäureethylester (2,0 g, 5,58 mmol) wurden in 10 ml THF gelöst. Nach Zugabe von Lithiumhydroxid-hydrat (1,64 g, 39,08 mmol) in 30 ml Wasser wurde insgesamt 22 h bei Raumtemperatur gerührt. THF wurde abdestilliert und die Wasserphase mit Essigsäureethylester extrahiert. Die Wasserphase wurde anschließend mit 4n Salzsäure angesäuert und das ausgefallene Produkt abgesaugt, mit Wasser gewaschen und getrocknet. Ausbeute: 1,64 g (89%), M+H+: 331,09.

### 2b: 2-(4-Phenoxy-phenyl)-3H-benzimidazol-5-carbonsäure-(3-pyridin-3-yl-propyl)-amid

Zu 2-(4-Phenoxy-phenyl)-3H-benzimidazol-5-carbonsäure (178 mg, 0,539 mmol) und 3-Pyridin-3-yl-propylamin (80,09 mg, 0,594 mmol) in 10 ml DMF wurden Triethylamin (0,15 ml, 1,078 mmol) und HATU (0,246 mg, 0,647 mmol) zugegeben und 6 h bei Raumtemperatur gerührt. Das Reaktionsgemisch wurde im Vakuum eingeengt, der Rückstand mit Wasser und Essigsäureethylester versetzt, die organisch Phase abgetrennt, mit Wasser gewaschen, getrocknet und eingeengt. Das erhaltene Rohprodukt wurde durch präparative HPLC (RP18, Acetonitril/Wasser 0,1% TFA) gereinigt. Ausbeute: 217 mg (90%), M+H+: 449,29.

### Beispiel 3:

2-(4-Phenoxy-phenyl)-3H-benzimidazol-5-carbonsäure-(3-phenyl-propyl)-amid Analog Beispiel 2b wurden 2-(4-Phenoxy-phenyl)-3H-benzimidazol-5-carbonsäure und 3-Phenyl-propylamin umgesetzt. Ausbeute: 97 mg (40%), M+H+: 448,28.

### Beispiel 4:

2-(4-Phenoxy-phenyl)-3H-benzimidazol-5-carbonsäure-phenethylamid Analog Beispiel 2b wurden 2-(4-Phenoxy-phenyl)-3H-benzimidazol-5-carbonsäure und Phenethylamin umgesetzt. Ausbeute: 70 mg (30%), M+H+: 434,26.

### Beispiel 5:

2-(4-Phenoxy-phenyl)-3 H-benzimidazol-5-carbonsäure-(3-methyl-butyl)-amid Analog Beispiel 2b wurden 2-(4-Phenoxy-phenyl)-3H-benzimidazol-5-carbonsäure und 3-Methyl-butylamin umgesetzt. Ausbeute: 58 mg (27%), M+H+: 400,27.

### Beispiel 6:

2-(4-Phenoxy-phenyl)-3H-benzimidazol-5-carbonsäure- (2-cyclopropyl-ethyl)-amid Analog Beispiel 2b wurden 2-(4-Phenoxy-phenyl)-3H-benzimidazol-5-carbonsäure und 2-Cyclopropyl-ethylamin-Hydrochlorid umgesetzt. Ausbeute: 26 mg (12%), M+H+: 398,16.

### Beispiel 7:

### 2-(4-Phenoxy-phenyl)-3H-benzimidazol-5-carbonsäure-cyclopropylmethyl-amid

Analog Beispiel 2b wurden 2-(4-Phenoxy-phenyl)-3H-benzimidazol-5-carbonsäure und C-Cyclopropylmethyl-amin umgesetzt. Ausbeute: 74 mg (36%), M+H+: 384,14.

### Beispiel 8:

### 2-(4-Phenoxy-phenyl)-3H-benzimidazol-5-carbonsäre-(5-chloro-thiophen-2-ylmethyl)-amid

Analog Beispiel 2b wurden 2-(4-Phenoxy-phenyl)-3H-benzimidazol-5-carbonsäure und C-(5-Chlor-thiophen-2-yl)-methylamin umgesetzt. Ausbeute: 65 mg (26%), M+H+: 460,06.

### Beispiel 9:

### 2-(4-Phenoxy-phenyl)-3H-benzimidazol-5-carbonsäure-(thiazol-2-ylmethyl)-amid

Analog Beispiel 2b wurden 2-(4-Phenoxy-phenyl)-3H-benzimidazol-5-carbonsäure und C-(Thiazol-2-yl)-methylamin umgesetzt. Ausbeute: 84 mg (37%), M+H+: 427,09.

### Beispiel 10:

### 2-(4-Phenoxy-phenyl)-3H-benzimidazol-5-carbonsäure-(3-butoxy-propyl)-amid

Analog Beispiel 2b wurden 2-(4-Phenoxy-phenyl)-3H-benzimidazol-5-carbonsäure und 3-Butoxy-propylamin umgesetzt. Ausbeute: 111 mg (46%), M+H+: 444,17.

### Beispiel 11:

### 2-[4-(2-Chlor-phenoxy)-phenyl]-3H-benzimidazol-5-carbonsäreethylester

Analog Beispiel 1 wurden 4-(2-Chlor-phenoxy)-benzaldehyd und 3,4-Diaminobenzoesäureethylester umgesetzt. Ausbeute: 1,75 g (100%), M+H+: 393,17.

### Beispiel 12:

### 2-[4-(2-Chlor-phenoxy)-phenyl]-3H-benzoimidazol-5-carbonsäure-phenethyl-amid

### 12a: 2-[4-(2-Chlor-phenoxy)-phenyl]-3H-benzimidazol-5-carbonsäre

### Analog Beispiel 2a wurde 2-[4-(2-Chlor-phenoxy)-phenyl]-3H-benzimidazol-5-carbonsäureethylester umgesetzt. Ausbeute: 1,49 g (100%), M+H+: 365,08.

### 12b: 2-[4-(2-Chlor-phenoxy)-phenyl]-3H-benzoimidazol-5-carbonsäure-phenethyl-amid

Analog Beispiel 2b wurden 2-[4-(2-Chlor-phenoxy)-phenyl]-3H-benzimidazol-5-carbonsäure und Phenethylamin umgesetzt. Ausbeute: 65 mg (34%), M+H+: 468,36.

### Beispiel 13:

### 2-[4-(2-Chlor-phenoxy)-phenyl]-3H-benzoimidazol-5-carbonsäure-(3-methyl-butyl)-amid

Analog Beispiel 2b wurden 2-[4-(2-Chlor-phenoxy)-phenyl]-3H-benzimidazol-5-carbonsäure und 3-Methyl-butylamin umgesetzt. Ausbeute: 117 mg (66%), M+H+: 434,35.

### Beispiel 14:

### 2-[4-(2-Chlor-phenoxy)-phenyl]-3H-benzoimidazol-5-carbonsäure-(2-cyclopropyl-ethyl)-amid

Analog Beispiel 2b wurden 2-[4-(2-Chlor-phenoxy)-phenyl]-3H-benzimidazol-5-carbonsäure und 2-Cyclopropyl-ethylamin-Hydrochlorid umgesetzt. Ausbeute: 79 mg (45%), M+H+: 432,33.

### Beispiel 15:

### 2-[4-(2-Chlor-phenoxy)-phenyl]-3H-benzoimidazol-5-carbonsäure-(5-chloro-thiophen-2-ylmethyl)-amid

Analog Beispiel 2b wurden 2-[4-(2-Chlor-phenoxy)-phenyl]-3H-benzimidazol-5-carbonsäure und C-(5-Chlor-thiophen-2-yl)-methylamin umgesetzt. Ausbeute: 95 mg (47%), M+H+: 494,28.

### Beispiel 16:

### 2-[4-(2-Chlor-phenoxy)-phenyl]-3H-benzoimidazol-5-carbonsäure-(thiazol-2-ylmethyl)-amid

Analog Beispiel 2b wurden 2-[4-(2-Chlor-phenoxy)-phenyl]-3H-benzimidazol-5-carbonsäure und C-(Thiazol-2-yl)-methylamin umgesetzt. Ausbeute: 99 mg (52%), M+H+: 461,29.

### Beispiel 17:

### 2-[4-(2-Trifluormethyl-phenoxy)-phenyl]-3H-benzimidazole-5- carbonsäure-ethyl ester

### 17a: 4-(2-Trifluormethyl-phenoxy)-benzaldehyd

4-Fluorbenzaldehyd (397 mg, 3,2 mmol), 2-Hydroxybenzotrifluorid (1,14 g, 7,04 mmol) und Cäsiumcarbonat (1,04 g, 7,04 mmol) wurden in 20 ml DMF 6 h bei 90°C gerührt. Das Reaktionsgemisch wurde im Vakuum eingeengt und der Rückstand in Wasser und Essigsäureethylester aufgenommen, die organisch Phase abgetrennt, mit Wasser gewaschen, getrocknet und eingeengt. Das erhaltene Rohprodukt wurde durch präparative HPLC (RP18, Acetonitril/Wasser 0,1% TFA) gereinigt. Ausbeute: 108 mg (13%), M+H+: 267,15.

### 17b: 2-[4-(2-Trifluormethyl-phenoxy)-phenyl]-3H-benzimidazole-5-carbonsäure-ethyl ester

Zu einer Lösung von 4-(2-Trifluormethyl-phenoxy)-benzaldehyd (100 mg, 0,376 mmol) in 10 ml Ethanol wurden unter Argon Natriummetabisulfit (71,5 mg, 0,376 mmol) in 5 ml Wasser zugetropft. Das Reaktionsgemisch wurde 4 h bei Raumtemperatur gerührt und über Nacht im Kühlschrank aufbewahrt. Das Reaktionsgemisch wurde im Vakuum eingeengt, der Rückstand in 15 ml DMF gelöst und nach Zugabe von 3,4-Diaminobenzoesäureethylester (67,8 mg, 0,376 mmol) 6 h bei 130 °C gerührt. DMF wurde im Vakuum abdestilliert, der Rückstand mit Wasser und Essigsäureethylester versetzt, die organisch Phase abgetrennt, mit Wasser gewaschen, getrocknet und eingeengt. Das erhaltene Rohprodukt wurde durch präparative HPLC (RP18, Acetonitril/Wasser 0,1% TFA) gereinigt. Ausbeute: 24 mg (14%), M+H+: 427,08.

### Beispiel 18:

### 2-[4-(2-Trifluormethyl-phenoxy)-phenyl]-3H-benzimidazole-5-carbonsäure-(5-chlor-thiophen-2-ylmethyl)-amid

### 18a: 4-(2-Trifluormethyl-phenoxy)brombenzol

Kaliumhydroxid (5,06 g, 90 mmol) wurde in 58 ml einer Mischung aus DMSO/H2O 17/1 gelöst. Dazu wurde 4-Bromphenol (13,.5 g, 78 mmol) portionweise zugegeben und 5 Minuten unter Rühren auf 80 °C erwärmt. Anschließend wurde 1-Fluoro-2-trifluormethyl-benzol (10 g, 61 mmol) schnell zugetropft und 2 h bei 120°C gerührt. Nach dem Abkühlen auf Raumtemperatur wurden 100 ml Wasser zugegeben. Die Wasserphase wurde 3 mal mit je 150 ml Diethylether extrahiert, die organischen Phasen wurden vereinigt, mit Wasser und gesättigter Natriumchlorid-Lösung gewaschen, über Magnesiumsulfat getrocknet und eingeengt. Ausbeute: 13,4 g (69%), M+H+: 317,2.

### 18b: 4-(2-Trifluormethyl-phenoxy)-benzaldehyd

Unter Inertgasatmosphare wurden 4-(2-Trifluormethyl-phenoxy)brombenzol (1,0 g, 3,15 mmol) in 20 ml wasserfreiem THF gelöst und auf -78°C abgekühlt. Unter Rühren wurden 6,31 ml einer Butyllithium-Lösung (2n in Hexan) zugetropft. Nach 5 Minuten wurden 1,46 ml DMF (18,9 mmol) zugegeben und die Lösung unter Rühren auf Raumtemperatur erwärmen lassen. Nach Zugabe von 10 ml Ammoniumchlorid-Lösung wurde mit Dichloromethan extrahiert, die organischen Phasen vereinigt gewaschen, über Magnesiumsulfat getrocknet und eingeengt. Der Rückstand wurde chromatographisch gereinigt (Kieselgel, Laufmittel Heptane/Esigsäureethylester 90:10). Ausbeute: 340 mg (15%), M+H+: 267,1.

### 18c: 2-[4-(2-Trifluormethyl-phenoxy)-phenyl]-3H-benzimidazole-5-carbonsäure

Zu einer Lösung von 4-(2-Trifluormethyl-phenoxy)-benzaldehyd (0,74 g, 2,78 mmol) in 3 ml DMF wurden 3,4-Diaminobenzoesäure (0,465 g, 3,06 mmol) und Natriummetabisulfit (0,.67g, 3,.612 mmol) zugegeben. Die Mischung wurde 5 min in einem Biotage® Mikrowellenreaktor bei 100 °C gerührt, abgekühlt , mit 20 ml Esigsäureethylester verdünnt, und die organische Phase 5 mal mit je 10 ml Wasser gewaschen, über Magnesiumsulfat getrocknet und eingeengt. Ausbeute: 780 mg (70%), M+H+: 399,0.

### 18d: 2-[4-(2-Trifluormethyl-phenoxy)-phenyl]-3H-benzimidazole-5-carbonsäure-(5-chlorthiophen-2-ylmethyl)-amid

Zu 2-[4-(2-Trifluormethyl-phenoxy)-phenyl]-3H-benzimidazole-5-carbonsäure (0,1793 g, 0.45 mmol) in 2 ml Acetonitril wurden 1,3-Diethylcarbodiimid (172,.5 mg, 0,9 mmol), HOBT (172,.5 mg, 0,9 mmol), Ttriethylamin (0,25 mL) and (5-Chloro-thiophen-2-ylmethyl)-amin (82,8 mg, 0,45 mmol) gegeben. Das Reaktionsgemisch wurde über Nacht bei Raumtemperatur gerührt, mit 20 ml Dichloromethan versetzt, mit 10 ml Wasser gewaschen und die organische Phase über Magnesiumsulfat getrocknet und eingeengt. Der Rückstand wurde chromatographisch gereinigt (Kieselgel, Laufmittelgradient Heptane/ Esigsäureethylester 94:6 bis 40:60). Ausbeute: 87 mg (37%), M+H+: 528,0.

### Beispiel 19:

### 2-[4-(2-Trifluormethyl-benzoyl)-phenyl]-3H-benzimidazole-5-carbonsäure-(thiophen-2-ylmethyl)-amid

### 19a: 4-(2-Trifluormethyl-benzoyl)-benzaldehyd

Zu (4-[1,3]Dioxan-2-yl-phenyl)-(2-trifluormethyl-phenyl)-methanon (2,0 g, 5,95mmol) in 13 ml einer Dioxan/Wasser-Mischung wurden 4,43 ml einer 4M Salzsäurelösung gegeben und 60 h bei Raumtemperatur gerührt. Nach dem Einengen der Lösungsmittel im Vakuum wurde mit Dichloromethan extrahiert, die organische Phase eingeengt und über Kieselgel mit Dichloromethan als Laufmittel chromatographiert. Ausbeute: 1,31 g (79%), M+H+: 279,3.

### 19b: 3,4-Dinitro-N-thiophen-2-ylmethyl-benzamid

Zu 3,4-Dinitrobenzoesäure (1,0 g, 4,71 mmol) in 11 ml Methylenchlorid wurden bei -25 °C Oxalylchlorid (2,27 ml, 26,4 mmol) und ein Tropfen DMF zugegeben. Das Reaktionsgemisch wurde über Nacht bei Raumtemperatur gerührt, im Vakuum eingeengt. Der Rückstand wurde in 11 ml Dichloromethan gelöst und bei -5 °C Thiophen-2-ylmethyl-amin (0,56 g, 4,95 mmol) in Pyridin(1 mL) and Dichloromethan (10 mL)zugetropft. Das Reaktionsgemisch wurde 4 h bei Raumtemperatur gerührt, im Vakuum eingeengt.Das Rohprodukt wurde über Kieselgel mit Dichloromethan /Methanol 98:2 als Laufmittel chromatographiert. Ausbeute: 0,996 g (69%), M+H+: 308,0338.

### 19c: 3,4-Diamino-N-thiophen-2-ylmethyl-benzamid

3,4-Dinitro-N-thiophen-2-ylmethyl-benzamid (0,41 g, 1,33 mmol) wurden in 10 ml Ethanol/THF (1/1) in Gegenwart von 40 mg 10% Pd/C bei 2 h bei Raumtemperatur hydriert, über Celite filtriert und eingeengt. Ausbeute: 320 mg (97%), M+H+: 248,2.

### 19d: 2-[4-(2-Trifluormethyl-benzoyl)-phenyl]-3H-benzimidazole-5-carbonsäure-(thiophen-2-ylmethyl)-amid

3,4-Diamino-N-thiophen-2-ylmethyl-benzamid (100 mg, 0,4 mmol) und 4-(2-Trifluormethyl-benzoyl)-benzaldehyd (110 mg, 0,4 mmol) wurden in 8 ml Dioxan mit Diacetoxyiodbenzol (191 mg, 0,.59 mmol) 2 h bei Raumtemperatur gerührt. Dann wurde nochmals Diacetoxyiodbenzol (191 mg, 0,.59 mmol) zugegeben, über Nacht bei Raumtemperatur gerührt und eingeengt. Der Rückstand wurde chromatographisch gereinigt (Kieselgel, Laufmittel Esigsäureethylester/ Cyclohexan 1:6). Ausbeute: 168 mg (84%), M+H+: 506,2.

### Beispiel 20:

### 2-[4-(2-Trifluormethyl-benzoyl)-phenyl]-3H-benzoimidazol-5-carbonsäure-isobutylamid

Die Verbindung wurde analog der oben beschriebenen Verfahren hergestellt.

### Beispiel 23:

### 2-[4-(2-Trifluormethyl-phenoxy)-phenyl]-3H-benzimidazol-5-r,arbonsäure-(2-cyclopropyl-ethyl)-amid

Die Verbindung wurde analog Beispiel 18d durch Umsetzung mit 2-Cyclopropyl-ethylamin erhalten. Ausbeute: 14%, M+H+: 466.

### Beispiel 24:

### 2-[4-(2-Trifluormethyl-phenoxy)-phenyl]-3H-benzimidazol-5-carbonsäure-(3-methyl-butyl)-amid

Die Verbindung wurde analog Beispiel 18d durch Umsetzung mit 3-Methyl-butylamin erhalten. Ausbeute: 25 %, M+H+: 468.

### Beispiel 25:

### 2-[4-(2-Trifluormethyl-phenoxy)-phenyl]-3H-benzimidazol-5-carbonsäure-(thiazol-2-ylmethyl)-amid

Die Verbindung wurde analog Beispiel 18d durch Umsetzung mit Thiazol-2-ylmethylamin erhalten. Ausbeute: 40%, M+H+: 495.

### Beispiel 26:

### 2-[4-(2-Trifluormethyl-phenoxy)-phenyl]-3H-benzimidazole5-carbonsäure-(3-phenyl-propyl)-amid

Die Verbindung wurde analog Beispiel 18d durch Umsetzung mit 3-Phenyl-propylamin erhalten. Ausbeute: 22%, M+H+: 516.

### Beispiel 27:

### 2-[4-(2-Trifluormethyl-benzoyl)-phenyl]-3H-benzimidazol-5-carbonsäure-5-chlor-thiophen-2-ylmethyl)-amid

### Beispiel 27a:

### 4-(2-Trifluormethyl-benzoyl)-benzaldehyd

(4-[1,3]dioxan-2-yl-phenyl)-(2-trifluormethyl-phenyl)-methanon (10.0g, 29.7 mmol) wurde in einer Mischung von 50 ml Essigsäure:Wasser 80:20 5,5 h bei 65 °C gerührt. Nach dem Abkühlen auf RT wurde die Reaktionsmischung in eine eiskalte, gesättigte Natriumbicarbonatlösung gegossen und dann mit Diethylether extrahiert. Die organische Phase wurde mit Wasser und gesättigter Kochsalzlösung gewaschen, über wasserfreiem Magnesiumsulfat getrocknet und im Vakuum eingeengt. Der Rückstand wurde über Säulenchromatographie mit einem Gradienten von Heptan: Dichloromethan 95:5 bis 50:50 als Laufmittel gereinigt. Nach dem entfernen der Lösungsmittel erhielt man 6,51 g 4-(2-Trifluormethyl-benzoyl)-benzaldehyd.

### Beispiel 27b:

### 2-[4-(2-Trifluormethyl-benzoyl)-phenyl]-1 H-benzimidazol-5-carbonsäure

Zu einer Lösung von 4-(2-Trifluormethyl-benzoyl)-benzaldehyd (3.0g, 10.78 mmol) in 18 mL DMF wurden unter Rühren 3,4-Diamino-benzoesäure(1.8g, 11.86 mmol) und Na₂S₂O₅ (2.66g, 14.02 mmol)zugegeben. Das Reaktionsgemisch wurde im Mikrowellenreaktor 3 mal 30 Minuten auf 100 °C erwärmt und dann filtriert. Das Filtrat wurde im Vakuum eingeengt und der ölige Rückstand durch Flashchromatographie mit einem Gradienten aus CH₂Cl₂ : MeOH : AcOH von 99.2:0.8:0.08 bis 92:8:0.8 gereinigt. Nach dem Einengen erhielt man 3.49g 2-[4-(2-Trifluormethyl-benzoyl)-phenyl]-1H-benzimidazol-5-carbonsäure. M+II+: 411.

### Beispiel 27c:

Die Verbindung wurde analog Beispiel 18d durch Umsetzung von 2-[4-(2-Trifluormethyl-benzoyl)-phenyl]-1H-benzimidazol-5-carbonsäure mit 5-Chlor-thiophen-2-ylmethylamin erhalten. Ausbeute: 41 %, M+H+: 540.

### Beispiel 28:

### 2-[4-(2-Trifluormethyl-benzoyl)-phenyl]-1H-benzimidazol-5-carbonsäure-(thiazol-2-ylmethyl)-amid

Die Verbindung wurde analog Beispiel 27c durch Umsetzung mit Thiazol-2-yl-methylamin erhalten. Ausbeute: 19%, M+H+: 507.

Bei den Beispielen 1, 11 und 17 handelt es sich um Zwischenprodukte, die zur Herstellung der Verbindungen der Formel I verwendet werden können.

## Patentansprüche

1. Verbindungen der Formel I, 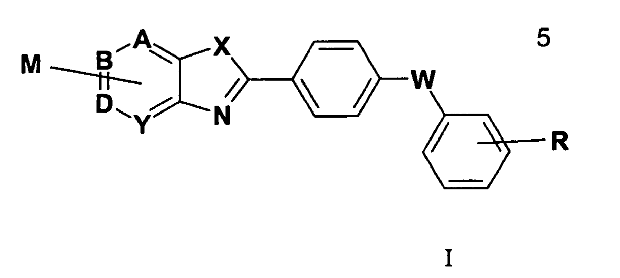 worin bedeuten
M R1-NH-C(=O)-;
W O, C=O;
X NH;
A, B, D, Y CH;
R (C₁-C₁₆)-Alkyl, (C₁-C₅)-Alkyloxy, (C₁-C₅)-Alkylthio, Amino, (C₁-C₅)- Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
R1 -(CH₂)ₙ-(C₆-C₁₀)-Aryl, -(CH₂)ₙ-(C₃-C₁₂)-Heteroaryl, -(CH₂)ₙ-(C₃-C₁₂)-Hetero- cyclyl oder -(CH₂)ₙ -(C₃-C₁₂)-Cycloalkyl,
wobei Aryl-, Heteroaryl-, Heterocyclyl- oder Cycloalkylringe gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
n 1, 2, 3, 4;
und deren physiologisch verträgliche Salze.

2. Verbindungen gemäß Anspruch 1, zur Anwendung als Arzneimittel.

3. Arzneimittel enthaltend eine oder mehrere der Verbindungen gemäß Anspruch 1.

4. Arzneimittel enthaltend eine oder mehrere der Verbindungen gemäß Anspruch 1 und mindestens einen weiteren Wirkstoff.

5. Arzneimittel, gemäß Anspruch 4, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

6. Verwendung der Verbindungen gemäß Anspruch 1 zur Herstellung eines Medikamentes zur Behandlung von Obesitas.

7. Verwendung der Verbindungen gemäß Anspruch 1 zur Herstellung eines Medikamentes zur Behandlung des Diabetes.

8. Verwendung der Verbindungen gemäß Anspruch 1 zur Herstellung eines Medikamentes zur Lipidsenkung.

9. Verwendung der Verbindungen gemäß Anspruch 1 zur Herstellung eines Medikamentes zur Behandlung des metabolischen Syndroms.

10. Verwendung der Verbindungen gemäß Anspruch 1 zur Herstellung eines Medikaments zur Behandlung von Insulin Resistenz.

11. Verwendung der Verbindungen gemäß Anspruch 1 zur Herstellung eines Medikamentes zur Behandlung von Herkreislauferkrankungen.

12. Verwendung der Verbindungen gemäß Anspruch 1 zur Herstellung eines Medikamentes zur Behandlung von ZNS-Erkrankungen.

13. Verwendung der Verbindungen gemäß Anspruch 1 zur Herstellung eines Medikamentes zur Behandlung von Schizophrenie.

14. Verwendung der Verbindungen gemäß Anspruch 1 zur Herstellung eines Medikamentes zur Behandlung von Alzheimer.

15. Verfahren zur Herstellung eines Arzneimittels enthaltend eine oder mehrere der Verbindungen gemäß Anspruch 1, **dadurch gekennzeichnet, dass** der Wirkstoff mit einem pharmazeutisch geeigneten Träger vermischt wird und diese Mischung in eine für die Verabreichung geeignete Form gebracht wird.

16. Verwendung der Verbindungen der Formel I, 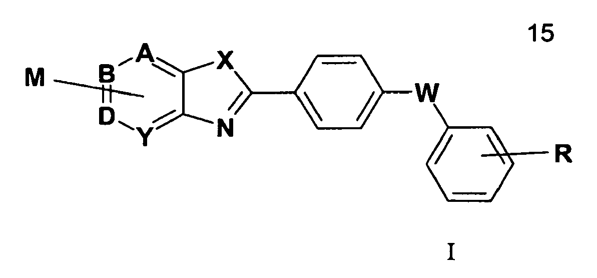 worin bedeuten
M R1-N(R2)-C(=O)-, R5-C(=O)-N(R1)-, R5-S(O)₀₋₂-N(R1)-, R1-N(R2)-S(O)₀₋₂-, ein Heterocyclus, der 2 bis 4 Heteroatome aus der Gruppe N, O, S enthalten kann, wobei der Heterocyclus substituiert sein kann durch (C₁-C₁₆)-Alkyl, oder doppelt gebundenen Sauerstoff;
W O, CH₂, C=O;
X N-R4, O, S;
A, B, D, Y unabhängig voneinander C(R3) oder N,
wobei maximal zwei der Reste A, B, D, Y die Bedeutung N haben können;
R Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
R1 (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Arryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
-(C₆-C₁₀)-Aryl, -(C₃-C₁₂)-Heteroaryl, -(C₃-C₁₂)-Heterocyclyl oder -(C₃-C₁₂)- Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁-C₆)-Alkylamino, Di-(C₂- C₁₂)-alkylamino, substituiert sein können;
R2 Wasserstoff, (C₂-C₁₆)-Alkyl, -(C₆-C₁₀)-aryl, (C₁-C₄)-Alkylen-(C₆-C₁₀)-aryl;
oder R1 und R2 bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, - C(=O)-, -O-, -S-, -SO- und -SO₂- ersetzt sein können;
R3 Wasserstoff, (C₁-C₆)-Alkyl, (C₁-C₃)-Alkyloxy, Hydroxy, (C₁-C₆)-Alkyl- mercapto, Amino, (C₁-C₆)-Alkylamino, Di-(C₂-C₁₂)-alkylamino, Cyano, (C₁- C₆)-Alkylcarbonyl, Halogen, Trifluormethyl, Trifluormethyloxy, (C₁-C₆)- Alkylsulfonyl, Aminosulfonyl;
R4 Wasserstoff, (C₁-C₅)-Alkyl;
R5 Wasserstoff, (C₁-C₁₆)-Alkyl, Amino, (C₁-C₁₆)-Alkylamino, Di-(C₂-C₁₂)- alkylamino,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
-(C₆-C₁₀)-Aryl, -(C₃-C₁₂)-Heteroaryl, -(C₃-C₁₂)-Heterocyclyl oder -(C₃-C₁₂)- Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁-C₆)-Alkylamino, Di-(C₂- C₁₂)-alkylamino, substituiert sein können;
und deren physiologisch verträgliche Salze zur Herstellung eines Medikamentes zur Behandlung von Obesitas.

17. Verwendung der Verbindungen der Formel I, gemäß Anspruch 16, **dadurch gekennzeichnet, dass** darin bedeuten
M R1-N(R2)-C(=O)-, R5-C(=O)-N(R1)-, R5-S(O)₀₋₂-N(R1)-, R1-N(R2)-S(O)₀₋₂-, ein Heterocyclus, der 2 bis 4 Heteroatome aus der Gruppe N, O, S enthalten kann, wobei der Heterocyclus substituiert sein kann durch (C₁-C₁₆)-Alkyl, oder doppelt gebundenen Sauerstoff;
W O, C=O;
X N-R4;
A, B, D, Y unabhängig voneinander C(R3) oder N,
wobei maximal zwei der Reste A, B, D, Y die Bedeutung N haben können;
R Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
R1 (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₂-C₁₆)-Alkyl, -(C₆-C₁₀)-aryl, (C₁-C₄)-Alkylen-(C₆-C₁₀)-aryl;
oder R1 und R2 bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, - C(=O)-, -O-, -S-, -SO- und -SO₂- ersetzt sein können;
R3 Wasserstoff, (C₁-C₆)-Alkyl, (C₁-C₃)-Alkyloxy, Hydroxy, (C₁-C₆)-Alkyl- mercapto, Amino, (C₁-C₆)-Alkylamino, Di-(C₂-C₁₂)-alkylamino, Cyano, (C₁- C₆)-Alkylcarbonyl, Halogen, Trifluormethyl, Trifluormethyloxy, (C₁-C₆)- Alkylsulfonyl, Aminosulfonyl;
R4 Wasserstoff, (C₁-C₅)-Alkyl
R5 Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)-Alkylamino, Di- (C₂-C₁₂)-alkylamino, -(C₆-C₁₀)-Aryl, -(C₃-C₁₂)-Heteroaryl, -(C₃-C₁₂)-Hetero- cyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)- Alkylmercapto, Amino, (C₁-C₆)-Alkylamino, Di-(C₂-C₁₂)-alkylamino, substituiert sein können;
und deren physiologisch verträgliche Salze zur Herstellung eines Medikamentes zur Behandlung von Obesitas.

18. Verwendung der Verbindungen der Formel I, gemäß Anspruch 16, **dadurch gekennzeichnet, dass** darin bedeuten
M R1-N(R2)-C(=O)-, R5-C(=O)-N(R1)-;
W O, C=O;
X N-R4;
A, B, D, Y wobei maximal unabhängig voneinander C(R3) oder N, zwei der Reste A, B, D, Y die Bedeutung N haben können;
R Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
R1 (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heteroeyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C_{1$}- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₂-C₁₆)-Alkyl, -(C₆-C₁₀)-aryl, (C₁-C₄)-Alkylen-(C₆-C₁₀)-aryl;
oder R1 und R2 bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, -CR4R5-, -(C=R4)-, -NR6-, - C(=O)-, -O-, -S-, -SO- und -SO₂- ersetzt sein können;
R3 Wasserstoff, (C₁-C₆)-Alkyl, (C₁-C₃)-Alkyloxy, Hydroxy, (C₁-C₆)-Alkyl- mercapto, Amino, (C₁-C₆)-Alkylamino, Di-(C₂-C₁₂)-alkylamino, Cyano, (C₁- C₆)-Alkylcarbonyl, Halogen, Trifluormethyl, Trifluormethyloxy, (C₁-C₆)- Alkylsulfonyl, Aminosulfonyl;
R4 Wasserstoff, (C₁-C₅)-Alkyl
R5 Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
und deren physiologisch verträgliche Salze zur Herstellung eines Medikamentes zur Behandlung von Obesitas.

19. Verwendung der Verbindungen der Formel I, gemäß Anspruch 16, **dadurch gekennzeichnet, dass** darin bedeuten
M R1-N(R2)-C(=O)-, RS-C(=O)-N(R1)-;
W O, C=O;
X N-R4;
A, B, D, Y CH;
R Wasserstoff, (C₁-C₁₆)-Alkyl, (C₁-C₅)-Alkyloxy, (C₁-C₅)-Alkylthio, Amino, (C₁- C₅)-Alkylamino, Di-(C₂-C₈)-alkylamino, (C₁-C₆)-Alkylcarbonyl-amino, (C₁-C₆)- Alkoxycarbonyl-amino, Halogen, Hydroxy, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl;
R1 (C₂-C₁₀)-Alkyl,
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
(C₁-C₁₀)-Alkyl,
wobei Alkyl substituiert ist mit -(C₆-C₁₀)-Aryl, -(C₃-C₁₂)-Heteroaryl, - (C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmereapto, Amino, (C₁- C₆)-Alkylamino, Di-(C₂-C₁₂)-alkylamino, substituiert sein können;
R2 Wasserstoff, (C₂-C₁₆)-Alkyl, -(C₆-C₁₀)-aryl, (C₁-C₄)-Alkylen-(C₆-C₁₀)-aryl;
oder R1 und R2 bilden zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem, deren einzelne Glieder der Ringsysteme durch ein bis drei Atome oder Atomgruppen aus der Gruppe -CHR4-, - CR4R5-, -(C=R4)-, -NR6-, -C(=O)-, -O-, -S-, -SO- und -SO₂- ersetzt sein können;
R4 Wasserstoff;
R5 Wasserstoff, (C₁-C₁₆)-Alkyl, Amino, (C₁-C₁₆)-Alkylamino, Di-(C₂-C₁₂)- alkylamino
wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₃-C₁₂)- Heteroaryl, -(C₃-C₁₂)-Heterocyclyl oder -(C₃-C₁₂)-Cycloalkyl,
wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
und deren physiologisch verträgliche Salze zur Herstellung eines Medikamentes zur Behandlung von Obesitas.

20. Verwendung der Verbindungen der Formel I, gemäß einem oder mehreren der Ansprüche 16 bis 19 zur Herstellung eines Medikamentes zur Behandlung des Diabetes.

21. Verwendung der Verbindungen der Formel I, gemäß einem oder mehreren der Ansprüche 16 bis 19 zur Herstellung eines Medikamentes zur Lipidsenkung.

22. Verwendung der Verbindungen der Formel I, gemäß einem oder mehreren der Ansprüche 16 bis 19 zur Herstellung eines Medikamentes zur Behandlung des metabolischen Syndroms.

23. Verwendung der Verbindungen der Formel I, gemäß einem oder mehreren der Ansprüche 16 bis 19 zur Herstellung eines Medikaments zur Behandlung von Insulin Resistenz.

24. Verwendung der Verbindungen der Formel I, gemäß einem oder mehreren der Ansprüche 16 bis 19 zur Herstellung eines Medikamentes zur Behandlung von Herkreislauferkrankungen.

25. Verwendung der Verbindungen der Formel I, gemäß einem oder mehreren der Ansprüche 16 bis 19 zur Herstellung eines Medikamentes zur Behandlung von ZNS-Erkrankungen.

26. Verwendung der Verbindungen der Formel I, gemäß einem oder mehreren der Ansprüche 16 bis 19 zur Herstellung eines Medikamentes zur Behandlung von Schizophrenie.

27. Verwendung der Verbindungen der Formel I, gemäß einem oder mehreren der Ansprüche 16 bis 19 zur Herstellung eines Medikamentes zur Behandlung von Alzheimer.

## Claims

1. A compound of the formula I, 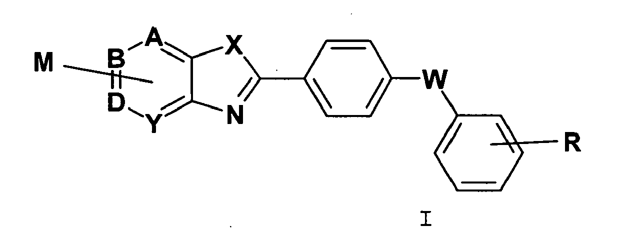 in which the meanings are
M R1-NH-C(=O)-;
W O, C=O;
X NH;
A, B, D, Y CH;
R (C₁-C₁₆)-alkyl, (C₁-C₅)-alkyloxy, (C₁-C₅)- alkylthio, amino, (C₁-C₅) -alkylamino, di- (C₂- C₈)-alkylamino, (C₁-C₆) -alkylcarbonylamino, (C₁-C₆)-alkoxycarbonylamino, halogen, hydroxy, mono-(C₁-C₆)-alkylaminocarbonyl, di-(C₂-C₈)- alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 -(CH₂)ₙ-(C₆-C₁₀)-aryl, -(CH₂)ₙ-(C₃-C₁₂)-heteroaryl, -(CH₂)ₙ-(C₃-C₁₂)-heterocyclyl or -(CH₂)ₙ -(C₃- C₁₂)-cycloalkyl,
where aryl, heteroaryl, heterocyclyl or cycloalkyl rings may optionally be substituted one or more times by halogen, (C₁-C₆)- alkyl, (C₁-C₃)-alkyloxy, hydroxy, (C₁-C₆)-alkylmercapto, amino, (C₁- C₆)-alkylamino, di- (C₂-C₁₂)- alkylamino;
n 1, 2, 3, 4;
and the physiologically tolerated salts thereof.

2. A compound as claimed in claim 1, for use as pharmaceutical.

3. A pharmaceutical comprising one or more of the compounds as claimed in claim 1.

4. A pharmaceutical comprising one or more of the compounds as claimed in claim 1 and at least one further active ingredient.

5. The pharmaceutical as claimed in claim 4, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, MTP inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-bisphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

6. The use of the compounds as claimed claim 1 for the manufacture of a medicament for the treatment of obesity.

7. The use of the compounds as claimed in claim 1 for the manufacture of a medicament for the treatment of diabetes.

8. The use of the compounds as claimed in claim 1 for the manufacture of a lipid-lowering medicament.

9. The use of the compounds as claimed in claim 1 for the manufacture of a medicament for the treatment of the metabolic syndrome.

10. The use of the compounds as claimed in claim 1 for the manufacture of a medicament for the treatment of insulin resistance.

11. The use of the compounds as claimed in claim 1 for the manufacture of a medicament for the treatment of cardiovascular disorders.

12. The use of the compounds as claimed in claim1 for the manufacture of a medicament for the treatment of CNS disorders.

13. The use of the compounds as claimed in claim 1 for the manufacture of a medicament for the treatment of schizophrenia.

14. The use of the compounds as claimed in claim 1 for the manufacture of a medicament for the treatment of Alzheimer's.

15. A process for the manufacture of a medicament comprising one or more of the compounds as claimed in claim 1, which comprises mixing the active ingredient with a pharmaceutically suitable carrier, and converting this mixture into a form suitable for administration.

16. The use of the compounds of the formula I 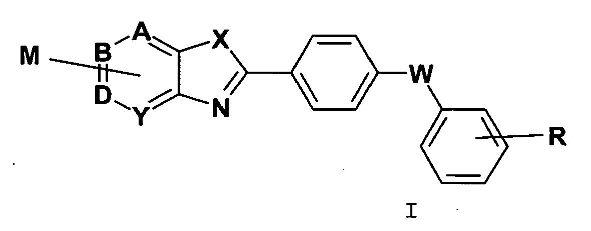 in which the meanings are
M R1-N(R2)-C(=O)-, R5-C(=O)-N(R1)-, R5-S(O)₀₋₂.- N(R1)-, R1-N(R2)-S(O)₀₋₂-, a heterocycle which may comprise 2 to 4 heteroatoms from the group of N, O, S, where the heterocycle may be substituted by (C₁-C₁₆)-alkyl, or doubly bonded oxygen;
W O CH₂, C=O;
X N-R4, O, S;
A, B, D, Y independently of one another C(R3) or N,
where a maximum of two of the radicals A, B, D, Y can have the meaning of N;
R hydrogen, (C₁-C₁₆)-alkyl, (C₁-C₅)-alkyloxy, (C₁- C₅)-alkylthio, amino, (C₁-C₅)-alkylamino, di- (C₂-C₈)-alkylamino, (C₁-C₆)-alkylcarbonylamino, (C₁-C₆)-alkoxycarbonylamino, halogen, hydroxy, mono-(C₁-C₆)-alkylaminocarbonyl, di-(C2-C8)- alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 (C₂-C₁₀)-alkyl,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, -(C₆-C₁₀)-aryl, -(C₃-C₁₂)- heteroaryl, -(C₃-C₁₂)-heterocyclyl or - (C₃-C₁₂)-cycloalkyl,
where aryl, heteroaryl, heterocyclyl or cycloalkyl may optionally be substituted one or more times by halogen, (C₁-C₆)-alkyl, (C₁-C₃)- alkyloxy, hydroxy, (C₁-C₆)- alkylmercapto, amino, (C₁-C₆)- alkylamino, di-(C₂-C₁₂)-alkylamino;
(C₁-C₁₀)-alkyl,
where alkyl is substituted by -(C₆-C₁₀) aryl, -(C₃-C₁₂)-heteroaryl, -(C₃-C₁₂)- heterocyclyl or -(C₃₋C₁₂₎-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
-(C₆-C₁₀)-aryl, -(C₃C₁₂)-heteroaryl, -(C₃-C₁₂)- heterocyclyl or -(C₃-C₁₂)-cycloalkyl,
where aryl, heteroaryl, heterocyclyl or cycloalkyl may optionally be substituted one or more times by halogen, (C₁-C₆)- alkyl, (C₁-C₃-alkyloxy, hydroxy, (C₁-C₆)- alkylmercapto, amino, (C₁-C₆)-alkylamino, di-(C₂-C₁₂)-alkylamino;
R2 hydrogen, (C₂-C₁₆)-alkyl, -(C₆-C₁₀)-aryl, (C₁-C₄)- alkylene-(C₆-C₁₀)-aryl;
or R1 and R2 form together with the nitrogen atom carrying them a monocyclic, saturated or partly unsaturated 4- to 7-membered ring system or a bicyclic saturated or partly unsaturated 8- to 14 membered ring system whose individual members of the ring system may be replaced by one to three atoms or atomic groups from the group of -CHR4-, -CR4R5-, -(C=R4)-, -NR6-, -C(=O)-, -O-, -S-, -SO- and -SO₂-;
R3 hydrogen, (C₁-C₆)-alkyl , (C₁-C3)-alkyloxy, hydroxy, (C₁-C₆)-alkylmercapto, amino, (C₁-C₆)- alkylamino, di-(C₂ -C₁₂)-alkylamino, cyano, (C₁- C₆)-alkylcarbonyl, halogen, trifluoromethyl, trifluoromethyloxy, (C₁-C₆)-alkylsulfonyl, aminosulfonyl;
R4 hydrogen, (C₁-C₅)-alkyl;
R5 hydrogen, (C₁-C₁₆) -alkyl, amino, (C₁-C₁₆)- alkylamino, di-(C₂-C₁₂)-alkylamino,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, -(C₆-C₁₀)-aryl, -(C₃-C₁₂)- heteroaryl, -(C₃-C₁₂)-heterocyclyl or - (C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di- (C₂-C₁₂)- alkylamino;
-(C₆-C₁₀)-aryl, -(C₃-C₁₂)-heteroaryl, -(C₃-C₁₂)- heterocyclyl or -(C₃-C₁₂₎-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)- alkylmercapto, amino, (C₁-C₆)-alkylamino, di- (C₂-C₁₂)-alkylamino;
and the physiologically tolerated salts thereof, for the manufacture of a medicament for the treatment of obesity.

17. The use of the compounds of the formula I as claimed in claim 16, wherein the meanings are
M R1-N(R2)-C(=O)-, R5-C(=O)-N(R1)-, R5-S(O)₀₋₂- N(R1)-, R1-N(R2)-S(O)₀₋₂-, a heterocycle which may comprise 2 to 4 heteroatoms from the group of N, O, S, where the heterocycle may be substituted by (C₁-C₁₆)-alkyl, or doubly bonded oxygen;
W O, C=O;
X N-R4;
A, B, D, Y independently of one another C(R3) or N,
where a maximum of two of the radicals A, B, D, Y may have the meaning of N;
R hydrogen, (C₁-C₁₆)-alkyl, (C₁-C₅)-alkyloxy, (C₁- C₅)-alkylthio, amino, (C₁-C₅)-alkylamino, di- (C₂-C₈)-alkylamino, (C₁-C₆)-alkylcarbonylamino, (C₁-C₆)-alkoxycarbonylamino, halogen, hydroxy, mono-(C₁-C₆)-alkylaminocarbonyl, di-(C₂-C₈)- alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 (C₂-C₁₀)-alkyl,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, -(C₆-C₁₀)-aryl, -(C₃-C₁₂)- heteroaryl, -(C₃-C₁₂)-heterocyclyl or - (C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
(C₁-C₁₀)-alkyl,
where alkyl is substituted by -(C₆-C₁₀)- aryl, -(C₃-C₁₂)-heteroaryl, -(C₃-C₁₂)- heterocyclyl or -(C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
R2 hydrogen, (C₂-C₁₆)-alkyl, -(C₆-C₁₀)-aryl, (C₁-C₄)- alkylene-(C₆-C₁₀)-aryl;
or R1 and R2 form together with the nitrogen atom carrying them a monocyclic, saturated or partly unsaturated 4- to 7-membered ring system or a bicyclic saturated or partly unsaturated 8- to 14 membered ring system whose individual members of the ring systems may be replaced by one to three atoms or atomic groups from the group of -CHR4-, - CR4R5-, -(C=R4)-, -NR6-, -C(=O)-, -O-, -S-, - SO- and -SO₂-;
R3 hydrogen, (C₁-C₆)-alkyl, (C₁-C₃)-alkyloxy, hydroxy, (C₁-C₆)-alkylmercapto, amino, (C₁-C₆)- alkylamino, di- (C₂-C₁₂)-alkylamino, cyano, (C₁- C₆)-alkylcarbonyl, halogen, trifluoromethyl, trifluoromethyloxy, (C₁-C₆)-alkylsulfonyl, aminosulfonyl;
R4 hydrogen, (C₁-C₅)-alkyl
R5 hydrogen, (C₁-C₁₆)-alkyl, amino, (C₁-C₁₆)- alkylamino, di-(C₂-C₁₂)-alkylamino,
where alkyl may be substituted by halogen, (C₁-C₆)-alkyl, (C₁-C₃)-alkyloxy, hydroxy, (C₁- C₆)-alkylmercapto, amino, (C₁-C₆)-alkylamino, di- (C₂-C₁₂)-alkylamino, -(C₆-C₁₀)-aryl, -(C₃- C₁₂)-heteroaryl, -(C₃-C₁₂)-heterocyclyl or -(C₃-C₁₂)-cycloalkyl,
where aryl, heteroaryl, heterocyclyl or cycloalkyl may optionally be substituted one or more times by halogen, (C₁-C₆)-alkyl, (C₁- C₃)-alkyloxy, hydroxy, (C₁-C₆)-alkylmercapto, amino, (C₁-C₆)-alkylamino, di- (C₂-C₁₂)- alkylamino;
and the physiologically tolerated salts thereof, for the manufacture of a medicament for the treatment of obesity.

18. The use of the compounds of the formula I as claimed in claim 16, wherein the meanings are
M R1-N(R2)-C(=O)-, R5-C(=O)-N(R1)-;
W O, C=O;
X N-R4;
A, B, D, Y independently of one another C(R3) or N,
where a maximum of two of the radicals A, B, D, Y may have the meaning of N;
R hydrogen, (C₁-C₁₆)-alkyl, (C₁-C₅)-alkyloxy, (C₁- C₅)-alkylthio, amino, (C₁-C₅)-alkylamino, di- (C₂-C₈)-alkylamino, (C₁-C₆)-alkylcarbonylamino, (C₁-C₆)-alkoxycarbonylamino, halogen, hydroxy, mono- (C₁-C₆) -alkylaminocarbonyl, di- (C₂-C₈)- alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 (C₂-C₁₀)-alkyl,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, - (C₆-C₁₀)-aryl, - (C₃-C₁₂)- heteroaryl, -(C₃-C₁₂)-heterocyclyl or - (C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
(C₁-C₁₀)-alkyl,
where alkyl is substituted by -(C₆-C₁₀)- aryl, -(C₃-C₁₂)-heteroaryl, -(C₃-C₁₂)- heterocyclyl or -(C₃-C₁₂)-cycloalkyl,
where aryl, heteroaryl, heterocyclyl or cycloalkyl may optionally be substituted one or more times by halogen, (C₁-C₆)- alkyl, (C₁-C3)-alkyloxy, hydroxy, (C₁-C₆)-alkylmercapto, amino, (C₁-C₆)-alkylamino, di-(C₂-C₁₂)- alkylamino;
R2 hydrogen, (C₂-C₁₆)-alkyl, -(C₆-C₁₀)-aryl, (C₁-C₄)- alkylene- (C₆-C₁₀)-aryl;
or R1 and R2 form together with the nitrogen atom carrying them a monocyclic, saturated or partly unsaturated 4- to 7-membered ring system or a bicyclic saturated or partly unsaturated 8- to 14 membered ring system whose individual members of the ring systems may be replaced by one to three atoms or atomic groups from the group of -CHR4-, - CR4R5-, -(C=R4)-, -NR6-, -C(=O)-, -O-, -S-, - SO- and -SO₂-
R3 hydrogen, (C₁-C₆)-alkyl, (C₁-C₃) -alkyloxy, hydroxy, (C₁-C₆)-alkylmercapto, amino, (C₁-C₆)- alkylamino, di-(C₂-C₁₂)-alkylamino, cyano, (C₁- C₆)-alkylcarbonyl, halogen, trifluoromethyl, trifluoromethyloxy, (C₁-C₆)-alkylsulfonyl, aminosulfonyl;
R4 hydrogen, (C₁-C₅)-alkyl
R5 hydrogen, (C₁-C₁₆)-alkyl, amino, (C₁-C₁₆)- alkylamino, di-(C₂-C₁₂)-alkylamino,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, -(C₆-C₁₀)-aryl, -(C₃-C₁₂)- heteroaryl, -(C₃-C₁₂)-heterocyclyl or -(C₃-C₁₂)-cycloalkyl,
where aryl,heteroaryl, heterocyclyl or cycloalkyl may optionally be substituted one or more times by halogen, (C₁-C₆)-alkyl, (C₁- C₃)-alkyloxy, hydroxy, (C₁-C₆)-alkylmercapto, amino, (C₁-C₆)-alkylamino, di-(C₂-C₁₂)- alkylamino;
and the physiologically tolerated salts thereof, for the manufacture of a medicament for the treatment of obesity.

19. The use of the compounds of the formula I as claimed in claim 16, wherein the meanings are
M R1-N(R2)-C(=O)-, R5-C(=O)-N(R1)-;
W O, C=O;
X N-R4;
A, B, D, Y CH;
R hydrogen, (C₁-C₁₆)-alkyl, (C₁-C₅)-alkyloxy, (C₁- C₅)-alkylthio, amino, (C₁-C₅)-alkylamino, di- (C₂-C₈)-alkylamino, (C₁-C₆)-alkylcarbonylamino, (C₁-C₆)-alkoxycarbonylamino, halogen, hydroxy, mono-(C₁-C₆)-alkylaminocarbonyl, di-(C₂-C₈)- alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 (C₂-C₁₀)-alkyl,
where alkyl may be substituted by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)- alkylamino, -(C₆-C₁₀)-aryl, -(C₃-C₁₂)- heteroaryl, -(C₃-C₁₂)-heterocyclyl or - (C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
(C₁-C₁₀)-alkyl,
where alkyl is substituted by -(C₆-C₁₀)- aryl, -(C₃-C₁₂)-heteroaryl, -(C₃-C₁₂)- heterocyclyl or -(C₃-C₁₂)-cycloalkyl,
where aryl, heteroaryl, heterocyclyl or cycloalkyl may optionally be substituted one or more times by halogen, (C₁-C₆)- alkyl, (C₁-C₃)-alkyloxy, hydroxy, (C₁-C₆)-alkylmercapto, amino, (C₁-C₆)-alkylamino, di-(C₂-C₁₂)- alkylamino;
R2 hydrogen, (C₂-C₁₆)-alkyl, -(C₆-C₁₀)-aryl, (C₁-C₄)- alkylene-(C₆-C₁₀)-aryl;
or R1 and R2 form together with the nitrogen atom carrying them a monocyclic, saturated or partly unsaturated 4- to 7-membered ring system or a bicyclic saturated or partly unsaturated 8- to 14 membered ring system whose individual members of the ring systems may be replaced by one to three atoms or atomic groups from the group of -CHR4-, -CR4R5-, -(C=R4)-, -NR6-, -C(=O)-, -O-, -S-, -SO- and -SO₂-;
R4 hydrogen;
R5 hydrogen, (C₁-C₁₆)-alkyl, amino, (C₁-C₁₆)- alkylamino, di-(C₂-C₁₂)-alkylamino
where alkyl may be substituted by halogen, (C₁-C₆)=alkyl, (C₁-C₃)-alkyloxy, hydroxy, (C₁-C₆)-alkylmercapto, amino, (C₁-C₆)- alkylamino, di-(C₂-C₁₂)-alkylamino, -(C₆- C₁₀)-aryl, -(C₃-C₁₂)-heteroaryl, -(C₃-C₁₂)- heterocyclyl or -(C₃-C₁₂)-cycloalkyl,
where aryl, heteroaryl, heterocyclyl or cycloalkyl may optionally be substituted one or more times by halogen, (C₁-C₆)-alkyl, (C₁-C₃)-alkyloxy, hydroxy, (C₁-C₆)- alkylmercapto, amino, (C₁-C₆)- alkylamino, di-(C₂-C₁₂)-alkylamino;
and the physiologically tolerated salts thereof for the manufacture of a medicament for the treatment of obesity.

20. The use of the compounds of the formula I as claimed in one or more of claims 16 to 19 for the manufacture of a medicament for the treatment of diabetes.

21. The use of the compounds of the formula I as claimed in one or more of claims 16 to 19 for the manufacture of a lipid-lowering medicament.

22. The use of the compounds of the formula I as claimed in one or more of claims 16 to 19 for the manufacture of a medicament for the treatment of the metabolic syndrome.

23. The use of the compounds of the formula I as claimed in one or more of claims 16 to 19 for the manufacture of a medicament for the treatment of insulin resistance.

24. The use of the compounds of the formula I as claimed in one or more of claims 16 to 19 for the manufacture of a medicament for the treatment of cardiovascular disorders.

25. The use of the compounds of the formula I as claimed in one or more of claims 16 to 19 for the manufacture of a medicament for the treatment of CNS disorders.

26. The use of the compounds of the formula I as claimed in one or more of claims 16 to 19 for the manufacture of a medicament for the treatment of schizophrenia.

27. The use of the compounds of the formula I as claimed in one or more of claims 16 to 19 for the manufacture of a medicament for the treatment of Alzheimer's.

## Revendications

1. Composés de formule I, 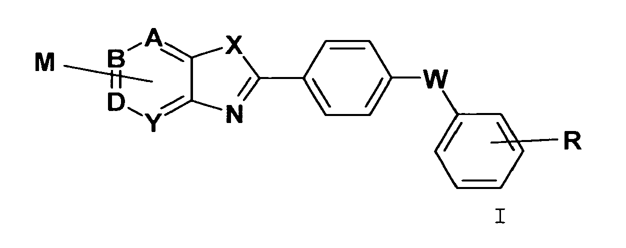 où
M signifie R1-NH-C(=O)- ;
W signifie O, C=O ;
X signifie NH ;
A, B, D, Y signifient CH ;
R signifie (C₁-C₁₆)-alkyle, (C₁-C₅)-alkyloxy, (C₁-C₅)- alkylthio, amino, (C₁-C₅) -alkylamino, di- (C₂-C₈)- alkylamino, (C₁-C₆)-alkylcarbonylamino, (C₁-C₆)- alcoxycarbonylamino, halogène, hydroxy, mono-(C₁- C₆)-alkylaminocarbonyle, di-(C₂-C₈)- alkylaminocarbonyle, (C₁-C₆)-alcoxycarbonyle, (C₁- C₆)-alkylcarbonyle, cyano, trifluorométhyle, trifluorométhyloxy, (C₁-C₆)-alkylsulfonyle ou aminosulfonyle ;
R1 signifie - (CH₂)ₙ-(C₆-C₁₀)-aryle, - (CH₂)ₙ-(C₃-C₁₂)- hétéroaryle, - (CH₂)ₙ-(C₃-C₁₂) -hétérocyclyle ou - (CH₂)ₙ-(C₃-C₁₂)-cycloalkyle,
où les cycles aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
n vaut 1, 2, 3, 4 ;
et leurs sels physiologiquement acceptables.

2. Composés selon la revendication 1, destinés à une utilisation comme médicament.

3. Médicament contenant un ou plusieurs des composés selon la revendication 1.

4. Médicament contenant un ou plusieurs des composés selon la revendication 1 et au moins une autre substance active.

5. Médicament selon la revendication 4, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'α-glucosidase, substances actives agissant sur le canal potassique dépendant d'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur-4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose 1 ou 2 dépendant du sodium, modulateurs de GPR40, inhibiteurs de la lipase sensible aux hormones, inhibiteurs de l'acétyl-CoA carboxylase, inhibiteurs de la phosphoénol-pyruvate-carboxykinase, inhibiteurs de la glycogène synthase kinase-3 bêta, inhibiteurs de la protéine kinase C bêta, antagonistes du récepteur de l'endothéline A, inhibiteurs de la I kappaB kinase, modulateurs du récepteur des glucocorticoïdes, agonistes de CART, antagonistes de NPY, agonistes de MC4, agonistes d'orexine, agonistes de H3, agonistes de TNF, agonistes de CRF, antagonistes de CRF BP, agonistes d'urocortine, agonistes de β3, antagonistes du récepteur de CB1, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR, agonistes de TR-β ou amphétamines.

6. Utilisation des composés selon la revendication 1 pour la préparation d'un médicament destiné au traitement de l'obésité.

7. Utilisation des composés selon la revendication 1 pour la préparation d'un médicament destiné au traitement du diabète.

8. Utilisation des composés selon la revendication 1 pour la préparation d'un médicament destiné à abaisser les lipides.

9. Utilisation des composés selon la revendication 1 pour la préparation d'un médicament destiné au traitement du syndrome métabolique.

10. Utilisation des composés selon la revendication 1 pour la préparation d'un médicament destiné au traitement de la résistance à l'insuline.

11. Utilisation des composés selon la revendication 1 pour la préparation d'un médicament destiné au traitement de maladies cardiovasculaires.

12. Utilisation des composés selon la revendication 1 pour la préparation d'un médicament destiné au traitement de maladie du SNC.

13. Utilisation des composés selon la revendication 1 pour la préparation d'un médicament destiné au traitement de la schizophrénie.

14. Utilisation des composés selon la revendication 1 pour la préparation d'un médicament destiné au traitement de la maladie d'Alzheimer.

15. Procédé pour la préparation d'un médicament contenant un ou plusieurs des composés selon la revendication 1, **caractérisé en ce que** la substance active est mélangée avec un support pharmaceutiquement approprié et ce mélange est amené dans une forme appropriée pour l'administration.

16. Utilisation des composés de formule I, 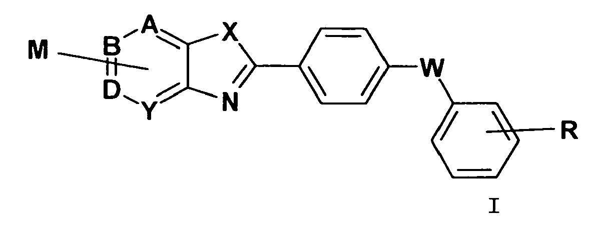 où
M signifie R1-N(R2)-C(=O)-, R5-C(=O)-N(R1)-, R5- S(O)₀₋₂-N(R1)-, R1-N(R2)-S(O)₀₋₂-, un hétérocycle, qui peut contenir 2 à 4 hétéroatomes du groupe formé par N, O, S, où l'hétérocycle peut être substitué par (C₁-C₁₆)-alkyle, ou de l'oxygène doublement lié ;
W signifie O, CH₂, C=O ;
X signifie N-R4, O, S ;
A, B, D, Y signifient, indépendamment les uns des autres, C(R3) ou N, où au maximum deux des radicaux A, B, D, Y peuvent signifier N ;
R signifie hydrogène, (C₁-C₁₆)-alkyle, (C₁-C₅)- alkyloxy, (C₁-C₅)-alkylthio, amino, (C₁-C₅)- alkylamino, di-(C₂-C₈)-alkylamino, (C₁-C₆)- alkylcarbonylamino, (C₁-C₆)-alcoxycarbonylamino, halogène, hydroxy, mono-(C₁-C₆)- alkylaminocarbonyle, di-(C₂-C₈)- alkylaminocarbonyle, (C₁-C₆)-alcoxycarbonyle, (C₁- C₆)-alkylcarbonyle, cyano, trifluorométhyle, trifluorométhyloxy, (C₁-C₆)-alkylsulfonyle ou aminosulfonyle ;
R1 signifie (C₂-C₁₀)-alkyle, où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, -(C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
signifie (C₁-C₁₀)-alkyle,
où alkyle est substitué par -(C₆-C₁₀)-aryle, -(C₃- C₁₂)-hétéroaryle, -(C₃-C₁₂)-hétérocyclyle ou -(C₃- C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
signifie -(C₆-C₁₀)-aryle, -(C₃-C₁₂)-hétéroaryle, -(C₃-C₁₂)-hétérocyclyle ou -(C₃-C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
R2 signifie hydrogène, (C₂-C₁₆)-alkyle, -(C₆-C₁₀)- aryle, (C₁-C₄)-alkylène-(C₆-C₁₀)-aryle ;
ou R1 et R2 forment ensemble avec l'atome d'azote qui les porte un système monocyclique saturé ou partiellement insaturé de 4 à 7 chaînons ou un système bicyclique saturé ou partiellement insaturé de 8 à 14 chaînons, les différents chaînons des systèmes cycliques pouvant être remplacés par un à trois atomes ou groupes d'atomes de la série -CHR4-, -CR4R5-, -(C=R4)-, -NR6-, -C(=O)-, -O-, -S-, -SO- et -SO₂- ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
R4 signifie hydrogène, (C₁-C₅)-alkyle ;
R5 signifie hydrogène, (C₁-C₁₆)-alkyle, amino, (C₁- C₁₆)-alkylamino, di-(C₂-C₁₂)-alkylamino,
où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₃- C₁₂)-hétéroaryle, -(C₃-C₁₂)-hétérocyclyle ou -(C₃-C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino ;
signifie -(C₆-C₁₀)-aryle, -(C₃-C₁₂)-hétéroaryle, -(C₃-C₁₂)-hétérocyclyle ou -(C₃-C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
et de leurs sels physiologiquement acceptables pour la préparation d'un médicament destiné au traitement de l'obésité.

17. Utilisation des composés de formule I selon la revendication 16, **caractérisée en ce que**
M signifie R1-N(R2)-C(=O)-, R5-C(=O)-N(R1)-, R5- S(O)₀₋₂-N(R1)-, R1-N(R2)-S(O)₀₋₂-, un hétérocycle, qui peut contenir 2 à 4 hétéroatomes du groupe formé par N, O, S, où l'hétérocycle peut être substitué par (C₁-C₁₆)-alkyle, ou de l'oxygène doublement lié ;
W signifie O, C=O ;
X signifie N-R4 ;
A, B, D, Y signifient, indépendamment les uns des autres, C(R3) ou N, où au maximum deux des radicaux A, B, D, Y peuvent signifier N ;
R signifie hydrogène, (C₁-C₁₆)-alkyle, (C₁-C₅)- alkyloxy, (C₁-C₅)-alkylthio, amino, (C₁-C₅)- alkylamino, di-(C₂-C₈)-alkylamino, (C₁-C₆)- alkylcarbonylamino, (C₁-C₆)-alcoxycarbonylamino, halogène, hydroxy, mono-(C₁-C₆)- alkylaminocarbonyle, di-(C₂-C₈)- alkylaminocarbonyle, (C₁-C₆)-alcoxycarbonyle, (C₁- C₆)-alkylcarbonyle, cyano, trifluorométhyle, trifluorométhyloxy, (C₁-C₆)-alkylsulfonyle ou aminosulfonyle ;
R1 signifie (C₂-C₁₀)-alkyle, où alkyle peut être substitué par halogène, (C₁- C₆)-alkyle, (C₁-C₃) -alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, -(C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
signifie (C₁-C₁₀)-alkyle,
où alkyle est substitué par -(C₆-C₁₀)-aryle, -(C₃-C₁₂)-hétéroaryle, -(C₃-C₁₂)-hétérocyclyle ou -(C₃-C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆) -alkylamino, di-(C₂-C₁₂)- alkylamino ;
R2 signifie hydrogène, (C₂-C₁₆)-alkyle, -(C₆-C₁₀)- aryle, (C₁-C₄)-alkylène-(C₆-C₁₀)-aryle ;
ou R1 et R2 forment ensemble avec l'atome d'azote qui les porte un système monocyclique saturé ou partiellement insaturé de 4 à 7 chaînons ou un système bicyclique saturé ou partiellement insaturé de 8 à 14 chaînons, les différents chaînons des systèmes cycliques pouvant être remplacés par un à trois atomes ou groupes d'atomes de la série -CHR4-, -CR4R5-, -(C=R4)-, -NR6-, -C(=O)-, -O-, -S-, -SO- et -SO₂- ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
R4 signifie hydrogène, (C₁-C₅)-alkyle
R5 signifie hydrogène, (C₁-C₁₆)-alkyle, amino, (C₁- C₁₆)-alkylamino, di-(C₂-C₁₂)-alkylamino,
où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, -(C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino ;
et de leurs sels physiologiquement acceptables pour la préparation d'un médicament destiné au traitement de l'obésité.

18. Utilisation des composés de formule I selon la revendication 16, **caractérisée en ce que**
M signifie R1-N(R2)-C(O)-, R5-C(=O)-N(R1)- ;
W signifie O, C=O ;
X signifie N-R4 ;
A, B, D, Y signifient, indépendamment les uns des autres, C(R3) ou N,
où au maximum deux des radicaux A, B, D, Y peuvent signifier N ;
R signifie hydrogène, (C₁-C₁₆)-alkyle, (C₁-C₅)- alkyloxy, (C₁-C₅)-alkylthio, amino, (C₁-C₅)- alkylamino, di-(C₂-C₈)-alkylamino, (C₁-C₆)- alkylcarbonylamino, (C₁-C₆)-alcoxycarbonylamino, halogène, hydroxy, mono-(C₁-C₆)- alkylaminocarbonyle, di-(C₂-C₈)- alkylaminocarbonyle, (C₁-C₆)-alcoxycarbonyle, (C₁- C₆)-alkylcarbonyle, cyano, trifluorométhyle, trifluorométhyloxy, (C₁-C₆)-alkylsulfonyle ou aminosulfonyle ;
R1 signifie (C₂-C₁₀)-alkyle, où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂) -alkylamino, -(C₆-C₁₀) -aryle, -(C₃C₁₂)- hétéroaryle, -(C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
signifie (C₁-C₁₀)-alkyle,
où alkyle est substitué par -(C₆-C₁₀)-aryle, -(C₃- C₁₂)-hétéroaryle, -(C₃-C₁₂)-hétérocyclyle ou -(C₃- C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
R2 signifie hydrogène, (C₂-C₁₆)-alkyle, -(C₆-C₁₀)- aryle, (C₁-C₄)-alkylène-(C₆-C₁₀)-aryle ;
ou R1 et R2 forment ensemble avec l'atome d'azote qui les porte un système monocyclique saturé ou partiellement insaturé de 4 à 7 chaînons ou un système bicyclique saturé ou partiellement insaturé de 8 à 14 chaînons, les différents chaînons des systèmes cycliques pouvant être remplacés par un à trois atomes ou groupes d'atomes de la série -CHR4-, -CR4R5-, -(C=R4)-, -NR6-, -C(=O)-, -O-, -S-, -SO- et -SO₂- ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
R4 signifie hydrogène, (C₁-C₅)-alkyle
R5 signifie hydrogène, (C₁-C₁₆)-alkyle, amino, (C₁- C₁₆)-alkylamino, di-(C₂-C₁₂)-alkylamino,
où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, (C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
et de leurs sels physiologiquement acceptables pour la préparation d'un médicament destiné au traitement de l'obésité.

19. Utilisation des composés de formule I selon la revendication 16, **caractérisée en ce que**
M signifie R1-N(R2)-C(=O)-, RS-C(O)-N(R1)- ;
W signifie O, C=O ;
X signifie N-R4 ;
A, B, D, Y signifie CH ;
R signifie hydrogène, (C₁-C₁₆)-alkyle, (C₁-C₅)- alkyloxy, (C₁-C₅)-alkylthio, amino, (C₁-C₅)- alkylamino, di-(C₂-C₈)-alkylamino, (C₁-C₆)- alkylcarbonylamino, (C₁-C₆)-alcoxycarbonylamino, halogène, hydroxy, mono-(C₁-C₆)- alkylaminocarbonyle, di-(C₂-C₈)- alkylaminocarbonyle, (C₁-C₆)-alcoxycarbonyle, (C₁- C₆)-alkylcarbonyle, cyano, trifluorométhyle, trifluorométhyloxy, (C₁-C₆)-alkylsulfonyle ou aminosulfonyle ;
R1 signifie (C₂-C₁₀)-alkyle, où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, -(C3-C12)-hétérocyclyle ou -(C₃-C12)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
signifie (C₁-C₁₀)-alkyle,
où alkyle est substitué par -(C₆-C₁₀)-aryle, -(C₃-C₁₂)-hétéroaryle, -(C₃-C₁₂)-hétérocyclyle ou -(C₃-C₁₂)-cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino ;
R2 signifie hydrogène, (C₂-C₁₆)-alkyle, -(C₆-C₁₀)- aryle, (C₁-C₄)-alkylène-(C₆-C₁₀)-aryle ;
ou R1 et R2 forment ensemble avec l'atome d'azote qui les porte un système monocyclique saturé ou partiellement insaturé de 4 à 7 chaînons ou un système bicyclique saturé ou partiellement insaturé de 8 à 14 chaînons, les différents chaînons des systèmes cycliques pouvant être remplacés par un à trois atomes ou groupes d'atomes de la série -CHR4-, -CR4R5-, -(C=R4)-, -NR6-, -C(=O)-, -O-, -S-, -SO- et -SO₂- ;
R4 signifie hydrogène ;
R5 signifie hydrogène, (C₁-C₁₆)-alkyle, amino, (C₁- C₁₆)-alkylamino, di-(C₂-C₁₂)-alkylamino, où alkyle peut être substitué par halogène, (C₁- C₆)-alkyle, (C₁-C₃)-alkyloxy, hydroxy, (C₁-C₆)- alkylmercapto, amino, (C₁-C₆)-alkylamino, di-(C₂- C₁₂)-alkylamino, -(C₆-C₁₀)-aryle, -(C₃-C₁₂)- hétéroaryle, -(C₃-C₁₂)-hétérocyclyle ou -(C₃-C₁₂)- cycloalkyle,
où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁-C₆)- alkylamino, di-(C₂-C₁₂)-alkylamino ;
et de leurs sels physiologiquement acceptables pour la préparation d'un médicament destiné au traitement de l'obésité.

20. Utilisation des composés de formule I, selon l'une ou plusieurs des revendications 16 à 19 pour la préparation d'un médicament destiné au traitement du diabète.

21. Utilisation des composés de formule I, selon l'une ou plusieurs des revendications 16 à 19 pour la préparation d'un médicament destiné à abaisser les lipides.

22. Utilisation des composés de formule I, selon l'une ou plusieurs des revendications 16 à 19 pour la préparation d'un médicament destiné au traitement du syndrome métabolique.

23. Utilisation des composés de formule I selon l'une ou plusieurs des revendications 16 à 19 pour la préparation d'un médicament destiné au traitement de la résistance à l'insuline.

24. Utilisation des composés de formule I, selon l'une ou plusieurs des revendications 16 à 19 pour la préparation d'un médicament destiné au traitement de maladies cardiovasculaires.

25. Utilisation des composés de formule I selon l'une ou plusieurs des revendications 16 à 19 pour la préparation d'un médicament destiné au traitement de maladies du SNC.

26. Utilisation des composés de formule I selon l'une ou plusieurs des revendications 16 à 19 pour la préparation d'un médicament destiné au traitement de la schizophrénie.

27. Utilisation des composés de formule I selon l'une ou plusieurs des revendications 16 à 19 pour la préparation d'un médicament destiné au traitement de la maladie d'Alzheimer.
